# EUROPEAN PATENT SPECIFICATION

(11) **EP 2 790 704 B1**
(45) Date of publication and mention of the grant of the patent: **03.04.2019**
(21) Application number: 12857229.4
(22) Date of filing: 12.12.2012
(51) Int. Cl.: A61K 31/513, A61K 38/28, A61P 3/10

(54) **TREATMENT OF TYPE I DIABETES**
BEHANDLUNG VON TYP-I-DIABETES
TRAITEMENT DU DIABÈTE DE TYPE I

(30) Priority: 12.12.2011 US 201161569496 P
(43) Date of publication of application: 22.10.2014
(73) Proprietor: Melior Pharmaceuticals I, Inc., Exton, PA 19341 (US)
(72) Inventor: REAUME, Andrew G., Exton, Pennsylvania 19341 (US); SAPORITO, Michael S., Exton, Pennsylvania 19341 (US); OCHMAN, Alexander R., Exton, Pennsylvania 19341 (US)
(74) Representative: Bassil, Nicholas Charles
(86) International application number: PCT/US2012/069072
(87) International publication number: WO 2013/090319

(56) References cited:
- WO-A1-2009/015133
- WO-A1-2011/150300
- US-A1- 2006 024 365
- US-A1- 2007 093 516
- US-A1- 2010 278 804
- CHARLES M E ST ET AL: "Health economic comparison between continuous subcutaneous insulin infusion and multiple daily injections of insulin for the treatment of adult type 1 diabetes in Canada", CLINICAL THERAPEUTICS, EXCERPTA MEDICA, PRINCETON, NJ, US, vol. 31, no. 3, 1 March 2009 (2009-03-01), pages 657-667, XP026021548, ISSN: 0149-2918, DOI: 10.1016/J.CLINTHERA.2009.03.013 [retrieved on 2009-03-01]

## Description

### Field

The present disclosure relates to methods of treating Type I or Type II diabetes in a mammal comprising administering insulin and a phenoxypyrimidinone compound, or pharmaceutically acceptable salts thereof, to the mammal, and to formulations thereto.

### Background

Diabetes is the most common disorder of the endocrine system and occurs when blood sugar levels in the body consistently stay above normal. It affects more than 23 million people in the U.S. alone. Diabetes is a disease brought on by either the body's inability to make insulin (Type I diabetes) or by the body's inability to respond to the effects of insulin (Type II diabetes). It can also appear during pregnancy. Insulin is one of the main hormones that regulates blood sugar levels and allows the body to use sugar for energy. Once Type II diabetes develops, symptoms include unusual thirst, a frequent need to urinate, blurred vision, or extreme fatigue. Type I diabetes occurs because the insulin-producing cells of the pancreas, beta cells, are destroyed by the immune system. People with Type I diabetes produce no insulin and must use insulin injections to control their blood sugar. Type I diabetes most commonly starts in people under the age of 20, but may occur at any age. Thus, compounds and compositions that can be used to treat Type I and/or Type II diabetes are clearly needed.

WO 2011/150300 describes compounds and pharmaceutically acceptable salts thereof and formulations that are useful in methods of preventing pancreatic beta cell degeneration or methods of treating a disorder associated with pancreatic beta cell degeneration, such as type I diabetes. WO 2009/015133 describes methods of activating IRS-1 and/or AKT and methods or treating or preventing IRS-1 and/or AKT related disease. St. Charles, M.E., et al. (Clin Ther. 2009; 31(3):657-667) describes a health economic comparison between continuous subcutaneous insulin infusion and multiple daily injections of insulin for the treatment of adult type 1 diabetes.

### Summary

The present invention provides a compound of Formula I: 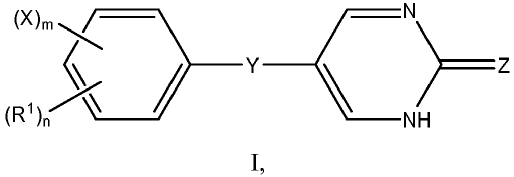 or a pharmaceutically acceptable salt thereof, wherein: R¹ is an alkyl group; X is a halogen; Y is O, S, or NH; Z is O or S; n is an integer from 0 to 5 and m is 0 or 1, wherein m + n is less than or equal to 5, for use in the treatment of Type I diabetes in a mammal wherein the treatment comprises a first administration of insulin followed by administration of the compound or pharmaceutically acceptable salt thereof.

In some embodiments the alkyl group is methyl n is 1. In some embodiments the halogen is chlorine and m is 1. In some embodiments Y is O. In some embodiments Z is O.

The compound for use according to the invention may be a compound of Formula II: 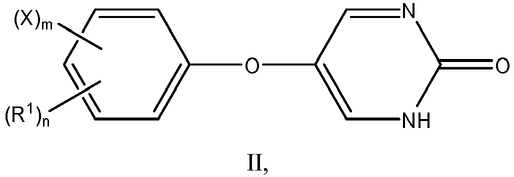 or a pharmaceutically acceptable salt thereof, wherein: R¹ is an alkyl group; X is a halogen; and n is an integer from 0 to 5 and m is 0 or 1, wherein m + n is less than or equal to 5.

The compound for use according to the invention may be a compound of Formula III: 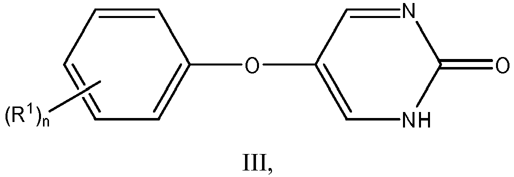 or a pharmaceutically acceptable salt thereof, wherein: R¹ is an alkyl group; and n is an integer from 0 to 5.

In some embodiments R¹ may be methyl and n is 1.

The compound according to the invention may be the compound: 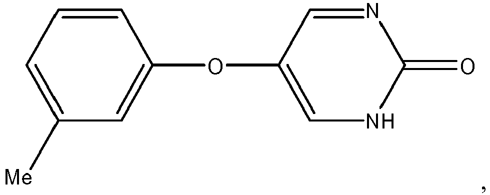 or a pharmaceutically acceptable salt thereof.

The compound for use according to the invention may be a compound of Formula IV: 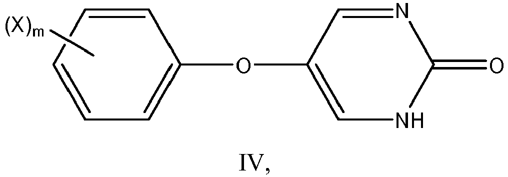 or a pharmaceutically acceptable salt thereof, wherein: X is a halogen; and m is 0 or 1.

In some embodiments X is a halogen and m is 0 or 1.

The compound for use according to the invention may be compound of formula VI: 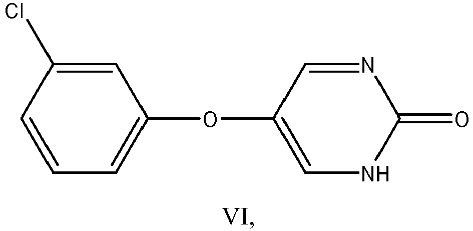 or a pharmaceutically acceptable salt thereof.

The amount of compound may be from about 0.1 mg/kg to about 100 mg/kg. The insulin may be selected from the group consisting of injectable insulin, transdermal insulin and inhaled insulin. The insulin may also be selected from the group consisting of rapid-acting insulin, short-acting insulin, intermediate-acting insulin and long-acting insulin. The amount of the rapid-acting insulin or short-acting insulin may be from about 0.05 U/kg/day to about 5 U/kg/day, from about 0.1 U/kg/day to about 2.5 U/kg/day, or from about 0.3 U/kg/day to about 0.8 U/kg/day. The amount of the intermediate acting insulin or long-acting insulin may be from about 0.01 U/kg/day to about 3 U/kg/day, from about 0.05 U/kg/day to about 0.6 U/kg/day, or from about 0.1 U/kg/day to about 0.3 U/kg/day.

Also described herein are formulations for oral administration in the form of a tablet, gel-cap, or capsule comprising insulin and from about 1 mg to about 1000 mg of the compound: 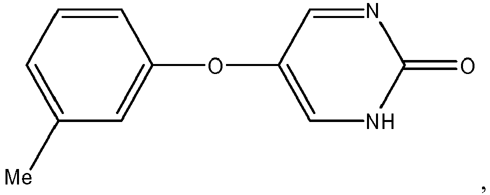 or a pharmaceutically acceptable salt thereof, wherein the formulation for oral administration is for use in treating or preventing Type I or Type II diabetes.

Also described herein are formulations for oral administration in the form of a tablet, gel-cap, or capsule comprising insulin and from about 1 mg to about 1000 mg of the compound: 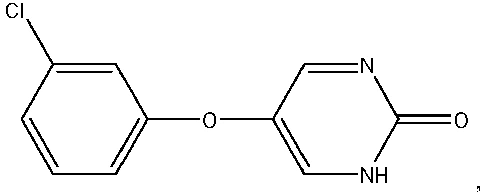 or a pharmaceutically acceptable salt thereof, wherein the formulation for oral administration is for use in treating or preventing Type I or Type II diabetes.

Also described herein is a composition comprising any one or more of the foregoing phenoxypyrimidinone compounds, or a pharmaceutically acceptable salt thereof, and insulin for treating Type I diabetes or Type II diabetes in a mammal.

Also described herein is a composition comprising any one or more of the foregoing phenoxypyrimidinone compounds, or a pharmaceutically acceptable salt thereof, and insulin for use in the manufacture of a medicament for treating Type I diabetes or Type II diabetes in a mammal.

Also described herein are uses of any one or more of the foregoing phenoxypyrimidinone compounds, or a pharmaceutically acceptable salt thereof, and insulin for treating Type I diabetes or Type II diabetes in a mammal.

Also described herein are uses of any one or more of the foregoing phenoxypyrimidinone compounds, or a pharmaceutically acceptable salt thereof, and insulin for use in the manufacture of a medicament for treating Type I diabetes or Type II diabetes in a mammal.

### Brief Description Of The Drawings

Figure 1 shows results of co-administration of the Compound of Formula IV with insulin enhanced the insulin blood glucose lowering response. *p < 0.05; comparing insulin to insulin/Compound co-administration.
Figure 2 shows results of co-administration of the Compound of Formula IV with insulin enhanced the insulin blood glucose lowering response. *p < 0.05; **p < 0.01 comparing insulin to insulin/Compound co-administration.
Figure 3 shows results of administration of the Compound of Formula IV potentiated and prolonged insulin-mediated blood glucose lowering. *p<0.05 when compared to insulin alone treatment. ***p<0.001 when compared to insulin alone treatment. Data are expressed as the average ± SEM. Data were analyzed by two-way ANOVA followed by post-hoc Bonferroni test.

### Description Of Disclosure

Unless defined otherwise, all technical and scientific terms have the same meaning as is commonly understood by one of ordinary skill in the art to which the disclosure belongs.

As used herein, the terms "a" or "an" means that "at least one" or "one or more" unless the context clearly indicates otherwise.

As used herein, the term "about" means that the numerical value is approximate and small variations would not significantly affect the practice of the disclosure. Where a numerical limitation is used, unless indicated otherwise by the context, "about" means the numerical value can vary by ±10% and remain within the scope of the disclosure.

As used herein, the term "alkenyl" means a straight or branched alkyl group having one or more double carbon-carbon bonds and 2-20 carbon atoms, including, but not limited to, ethenyl, 1-propenyl, 2-propenyl, 2-methyl-1-propenyl, 1-butenyl, 2-butenyl, and the like. As described herein, the alkenyl chain is from 2 to 10 carbon atoms in length, from 2 to 8 carbon atoms in length, from 2 to 6 carbon atoms in length, or from 2 to 4 carbon atoms in length.

As used herein, the term "alkoxy" means a straight or branched -O-alkyl group of 1 to 20 carbon atoms, including, but not limited to, methoxy, ethoxy, n-propoxy, isopropoxy, t-butoxy, and the like. As described herein, the alkoxy chain may be from 1 to 10 carbon atoms in length, from 1 to 8 carbon atoms in length, from 1 to 6 carbon atoms in length, from 1 to 4 carbon atoms in length, from 2 to 10 carbon atoms in length, from 2 to 8 carbon atoms in length, from 2 to 6 carbon atoms in length, or from 2 to 4 carbon atoms in length.

As used herein, the term "alkyl" means a saturated hydrocarbon group which is straight-chained or branched. An alkyl group can contain from 1 to 20, from 2 to 20, from 1 to 10, from 2 to 10, from 1 to 8, from 2 to 8, from 1 to 6, from 2 to 6, from 1 to 4, from 2 to 4, from 1 to 3, or 2 or 3 carbon atoms. Examples of alkyl groups include, but are not limited to, methyl (Me), ethyl (Et), propyl (e.g., n-propyl and isopropyl), butyl (e.g., n-butyl, t-butyl, isobutyl), pentyl (e.g., n-pentyl, isopentyl, neopentyl), hexyl, isohexyl, heptyl, 4,4-dimethylpentyl, octyl, 2,2,4-trimethylpentyl, nonyl, decyl, undecyl, dodecyl, 2-methyl-1-propyl, 2-methyl-2-propyl, 2-methyl-1-butyl, 3-methyl-1-butyl, 2-methyl-3-butyl, 2-methyl-1-pentyl, 2,2-dimethyl-1-propyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, and the like.

As used herein, the term "alkynyl" means a straight or branched alkyl group having one or more triple carbon-carbon bonds and 2-20 carbon atoms, including, but not limited to, acetylene, 1-propylene, 2-propylene, and the like. As described herein, the alkynyl chain may be 2 to 10 carbon atoms in length, from 2 to 8 carbon atoms in length, from 2 to 6 carbon atoms in length, or from 2 to 4 carbon atoms in length.

As used herein, the term "animal" includes, but is not limited to, humans and non-human vertebrates such as wild, domestic, and farm animals.

As used herein, the term "aryl" means a monocyclic, bicyclic, or polycyclic (e.g., having 2, 3 or 4 fused rings) aromatic hydrocarbons. As described herein, aryl groups have from 6 to 20 carbon atoms or from 6 to 10 carbon atoms. Examples of aryl groups include, but are not limited to, phenyl, naphthyl, anthracenyl, phenanthrenyl, indanyl, indenyl, tetrahydronaphthyl, and the like.

As used herein, the term "aryloxy" means an -O-aryl group, wherein aryl is as defined herein. An aryloxy group can be unsubstituted or substituted with one or two suitable substituents. The aryl ring of an aryloxy group can be a monocyclic ring, wherein the ring comprises 6 carbon atoms, referred to herein as "(C₆)aryloxy."

As used herein, the term "benzyl" means -CH₂-phenyl.

As used herein, the term "carbocycle" means a 5- or 6-membered, saturated or unsaturated cyclic ring, optionally containing O, S, or N atoms as part of the ring. Examples of carbocycles include, but are not limited to, cyclopentyl, cyclohexyl, cyclopenta-1,3-diene, phenyl, and any of the heterocycles recited above.

As used herein, the term "carrier" means a diluent, adjuvant, or excipient with which a compound is administered. Pharmaceutical carriers can be liquids, such as water and oils, including those of petroleum, animal, vegetable or synthetic origin, such as peanut oil, soybean oil, mineral oil, sesame oil and the like. The pharmaceutical carriers can also be saline, gum acacia, gelatin, starch paste, talc, keratin, colloidal silica, urea, and the like. In addition, auxiliary, stabilizing, thickening, lubricating and coloring agents can be used.

As used herein, the term, "compound" means all stereoisomers, tautomers, and isotopes of the compounds described herein.

As used herein, the terms "comprising" (and any form of comprising, such as "comprise", "comprises", and "comprised"), "having" (and any form of having, such as "have" and "has"), "including" (and any form of including, such as "includes" and "include"), or "containing" (and any form of containing, such as "contains" and "contain"), are inclusive or open-ended and do not exclude additional, unrecited elements or method steps.

As used herein, the term "cycloalkyl" means non-aromatic cyclic hydrocarbons including cyclized alkyl, alkenyl, and alkynyl groups that contain up to 20 ring-forming carbon atoms. Cycloalkyl groups can include mono- or polycyclic ring systems such as fused ring systems, bridged ring systems, and spiro ring systems. As described herein, polycyclic ring systems include 2, 3, or 4 fused rings. A cycloalkyl group can contain from 3 to 15, from 3 to 10, from 3 to 8, from 3 to 6, from 4 to 6, from 3 to 5, or 5 or 6 ring-forming carbon atoms. Ring-forming carbon atoms of a cycloalkyl group can be optionally substituted by oxo or sulfido. Examples of cycloalkyl groups include, but are not limited to, cyclopropyl, cyclobutyl, cyclopentyl, cyclohexyl, cycloheptyl, cyclooctyl, cyclononyl, cyclopentenyl, cyclohexenyl, cyclohexadienyl, cycloheptatrienyl, norbornyl, norpinyl, norcarnyl, adamantyl, and the like. Also included in the definition of cycloalkyl are moieties that have one or more aromatic rings fused (having a bond in common with) to the cycloalkyl ring, for example, benzo or thienyl derivatives of pentane, pentene, hexane, and the like (e.g., 2,3-dihydro-1H-indene-1-yl, or 1H-inden-2(3H)-one-1-yl).

As used herein, the term "diabetes" includes "Type I diabetes" and "Type II diabetes", and is often accompanied by related complications including, for example, obesity and high cholesterol.

As used herein, the term "halo" means halogen groups including, but not limited to fluoro, chloro, bromo, and iodo.

As used herein, the term "heteroaryl" means an aromatic heterocycle having up to 20 ring-forming atoms (e.g., C) and having at least one heteroatom ring member (ring-forming atom) such as sulfur, oxygen, or nitrogen. As described herein, the heteroaryl group may have at least one or more heteroatom ring-forming atoms, each of which are, independently, sulfur, oxygen, or nitrogen. As described herein, the heteroaryl group may have from 3 to 20 ring-forming atoms, from 3 to 10 ring-forming atoms, from 3 to 6 ring-forming atoms, or from 3 to 5 ring-forming atoms. As described herein, the heteroaryl group may contain 2 to 14 carbon atoms, from 2 to 7 carbon atoms, or 5 or 6 carbon atoms. As described herein, the heteroaryl group may have 1 to 4 heteroatoms, 1 to 3 heteroatoms, or 1 or 2 heteroatoms. Heteroaryl groups include monocyclic and polycyclic (e.g., having 2, 3 or 4 fused rings) systems. Examples of heteroaryl groups include, but are not limited to, pyridyl, pyrimidinyl, pyrazinyl, pyridazinyl, triazinyl, furyl, quinolyl, isoquinolyl, thienyl, imidazolyl, thiazolyl, indolyl (such as indol-3-yl), pyrryl, oxazolyl, benzofuryl, benzothienyl, benzthiazolyl, isoxazolyl, pyrazolyl, triazolyl, tetrazolyl, indazolyl, 1,2,4-thiadiazolyl, isothiazolyl, benzothienyl, purinyl, carbazolyl, benzimidazolyl, indolinyl, pyranyl, oxadiazolyl, isoxazolyl, triazolyl, thianthrenyl, pyrazolyl, indolizinyl, isoindolyl, isobenzofuranyl, benzoxazolyl, xanthenyl, 2H-pyrrolyl, pyrrolyl, 3H-indolyl, 4H-quinolizinyl, phthalazinyl, naphthyridinyl, quinazolinyl, phenanthridinyl, acridinyl, perimidinyl, phenanthrolinyl, phenazinyl, isothiazolyl, phenothiazinyl, isoxazolyl, furazanyl, phenoxazinyl groups, and the like. Suitable heteroaryl groups include 1,2,3-triazole, 1,2,4-triazole, 5-amino-1,2,4-triazole, imidazole, oxazole, isoxazole, 1,2,3-oxadiazole, 1,2,4-oxadiazole, 3-amino-1,2,4-oxadiazole, 1,2,5-oxadiazole, 1,3,4-oxadiazole, pyridine, and 2-aminopyridine.

As used herein, the term "heterocycle" or "heterocyclic ring" means a 5- to 7-membered mono- or bicyclic or 7- to 10-membered bicyclic heterocyclic ring system any ring of which may be saturated or unsaturated, and which consists of carbon atoms and from one to three heteroatoms chosen from N, O and S, and wherein the N and S heteroatoms may optionally be oxidized, and the N heteroatom may optionally be quaternized, and including any bicyclic group in which any of the above-defined heterocyclic rings is fused to a benzene ring. Particularly useful are rings containing one oxygen or sulfur, one to three nitrogen atoms, or one oxygen or sulfur combined with one or two nitrogen atoms. The heterocyclic ring may be attached at any heteroatom or carbon atom which results in the creation of a stable structure. Examples of heterocyclic groups include, but are not limited to, piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone, and oxadiazolyl. Morpholino is the same as morpholinyl.

As used herein, the term "heterocycloalkyl" means non-aromatic heterocycles having up to 20 ring-forming atoms including cyclized alkyl, alkenyl, and alkynyl groups, where one or more of the ring-forming carbon atoms is replaced by a heteroatom such as an O, N, or S atom. Hetercycloalkyl groups can be mono or polycyclic (e.g., fused, bridged, or spiro systems). As described herein, the heterocycloalkyl group may have from 1 to 20 carbon atoms, or from 3 to 20 carbon atoms. As described herein, the heterocycloalkyl group may contain 3 to 14 ring-forming atoms, 3 to 7 ring-forming atoms, or 5 or 6 ring-forming atoms. As described herein, the heterocycloalkyl group may have 1 to 4 heteroatoms, 1 to 3 heteroatoms, or 1 or 2 heteroatoms. As described herein, the heterocycloalkyl group may contain 0 to 3 double bonds. As described herein, the heterocycloalkyl group may contain 0 to 2 triple bonds. Examples of heterocycloalkyl groups include, but are not limited to, morpholino, thiomorpholino, piperazinyl, tetrahydrofuranyl, tetrahydrothienyl, 2,3-dihydrobenzofuryl, 1,3-benzodioxole, benzo-1,4-dioxane, piperidinyl, pyrrolidinyl, isoxazolidinyl, oxazolidinyl, isothiazolidinyl, pyrazolidinyl, thiazolidinyl, imidazolidinyl, pyrrolidin-2-one-3-yl, and the like. In addition, ring-forming carbon atoms and heteroatoms of a heterocycloalkyl group can be optionally substituted by oxo or sulfido. For example, a ring-forming S atom can be substituted by 1 or 2 oxo (form a S(O) or S(O)₂). For another example, a ring-forming C atom can be substituted by oxo (form carbonyl). Also included in the definition of heterocycloalkyl are moieties that have one or more aromatic rings fused (having a bond in common with) to the nonaromatic heterocyclic ring including, but not limited to, pyridinyl, thiophenyl, phthalimidyl, naphthalimidyl, and benzo derivatives of heterocycles such as indolene, isoindolene, isoindolin-1-one-3-yl, 4,5,6,7-tetrahydrothieno[2,3-c]pyridine-5-yl, 5,6-dihydrothieno[2,3-c]pyridin-7(4H)-one-5-yl, and 3,4-dihydroisoquinolin-1(2H)-one-3yl groups. Ring-forming carbon atoms and heteroatoms of the heterocycloalkyl group can be optionally substituted by oxo or sulfido.

As used herein, the term "individual" or "patient," used interchangeably, means any animal, including mammals, such as mice, rats, other rodents, rabbits, dogs, cats, swine, cattle, sheep, horses, or primates, such as humans.

As used herein, the phrase "in need thereof' means that the animal or mammal has been identified as having a need for the particular method or treatment. As described herein, the identification can be by any means of diagnosis. In any of the methods and treatments described herein, the animal or mammal can be in need thereof.

As used herein, the phrase "integer from 1 to 5" means 1, 2, 3, 4, or 5.

As used herein, the term "isolated" means that the compounds described herein are separated from other components of either (a) a natural source, such as a plant or cell, such as a bacterial culture, or (b) a synthetic organic chemical reaction mixture, such as by conventional techniques.

As used herein, the term "mammal" means a rodent (i.e., a mouse, a rat, or a guinea pig), a monkey, a cat, a dog, a cow, a horse, a pig, or a human. As described herein, the mammal may be a human.

As used herein, the term "n-membered", where n is an integer, typically describes the number of ring-forming atoms in a moiety, where the number of ring-forming atoms is n. For example, pyridine is an example of a 6-membered heteroaryl ring and thiophene is an example of a 5-membered heteroaryl ring.

As used used herein, the phrase "optionally substituted" means that substitution is optional and therefore includes both unsubstituted and substituted atoms and moieties. A "substituted" atom or moiety indicates that any hydrogen on the designated atom or moiety can be replaced with a selection from the indicated substituent groups, provided that the normal valency of the designated atom or moiety is not exceeded, and that the substitution results in a stable compound. For example, if a methyl group is optionally substituted, then 3 hydrogen atoms on the carbon atom can be replaced with substituent groups.

As used herein, the phrase "pharmaceutically acceptable" means those compounds, materials, compositions, and/or dosage forms which are, within the scope of sound medical judgment, suitable for use in contact with tissues of humans and animals. As described herein, "pharmaceutically acceptable" means approved by a regulatory agency of the Federal or a state government or listed in the U.S. Pharmacopeia or other generally recognized pharmacopeia for use in animals, and more particularly in humans.

As used herein, the phrase "pharmaceutically acceptable salt(s)," includes, but is not limited to, salts of acidic or basic groups. Compounds that are basic in nature are capable of forming a wide variety of salts with various inorganic and organic acids. Acids that may be used to prepare pharmaceutically acceptable acid addition salts of such basic compounds are those that form non-toxic acid addition salts, i.e., salts containing pharmacologically acceptable anions including, but not limited to, sulfuric, thiosulfuric, citric, maleic, acetic, oxalic, hydrochloride, hydrobromide, hydroiodide, nitrate, sulfate, bisulfate, bisulfite, phosphate, acid phosphate, isonicotinate, borate, acetate, lactate, salicylate, citrate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, bicarbonate, malonate, mesylate, esylate, napsydisylate, tosylate, besylate, orthophoshate, trifluoroacetate, and pamoate (i.e., 1,1'-methylene-bis-(2-hydroxy-3-naphthoate)) salts. Compounds that include an amino moiety may form pharmaceutically acceptable salts with various amino acids, in addition to the acids mentioned above. Compounds that are acidic in nature are capable of forming base salts with various pharmacologically acceptable cations. Examples of such salts include, but are not limited to, alkali metal or alkaline earth metal salts and, particularly, calcium, magnesium, ammonium, sodium, lithium, zinc, potassium, and iron salts. The present invention also includes quaternary ammonium salts of the compounds described herein, where the compounds have one or more tertiary amine moiety.

As used herein, the term "phenyl" means -C₆H₅. A phenyl group can be unsubstituted or substituted with one, two, or three suitable substituents.

As used herein, the terms "prevention" or "preventing" mean a reduction of the risk of acquiring a particular disease, condition, or disorder.

As used herein, the term "purified" means that when isolated, the isolate contains at least 90%, at least 95%, at least 98%, or at least 99% of a compound described herein by weight of the isolate.

As used herein, the phrase "quaternary ammonium salts" means derivatives of the disclosed compounds with one or more tertiary amine moieties wherein at least one of the tertiary amine moieties in the parent compound is modified by converting the tertiary amine moiety to a quaternary ammonium cation via alkylation (and the cations are balanced by anions such as Cl⁻, CH₃COO⁻, and CF₃COO⁻), for example methylation or ethylation.

As used herein, the phrase "substantially isolated" means a compound that is at least partially or substantially separated from the environment in which it is formed or detected.

As used herein, the phrase "suitable substituent" or "substituent" means a group that does not nullify the synthetic or pharmaceutical utility of the compounds described herein or the intermediates useful for preparing them. Examples of suitable substituents include, but are not limited to: C₁-C₆alkyl, C₁-C₆alkenyl, C₁-C₆alkynyl, C₅-C₆aryl, C₁-C₆alkoxy, C₃-C₅heteroaryl, C₃-C₆cycloalkyl, C₅-C₆aryloxy, -CN, -OH, oxo, halo, haloalkyl, -NO₂, -CO₂H, -NH₂, -NH(C₁-C₈alkyl), -N(C₁-C₈alkyl)₂, -NH(C₆aryl), -N(C₅-C₆aryl)₂, -CHO, -CO(C₁-C₆alkyl), -CO((C₅-C₆)aryl), -CO₂((C₁-C₆)alkyl), and -CO₂((C₅-C₆)aryl). One of skill in art can readily choose a suitable substituent based on the stability and pharmacological and synthetic activity of the compounds described herein.

As used herein, the phrase "therapeutically effective amount" means the amount of active compound or pharmaceutical agent that elicits the biological or medicinal response that is being sought in a tissue, system, animal, individual or human by a researcher, veterinarian, medical doctor or other clinician. The therapeutic effect is dependent upon the disorder being treated or the biological effect desired. As such, the therapeutic effect can be a decrease in the severity of symptoms associated with the disorder and/or inhibition (partial or complete) of progression of the disorder, or improved treatment, healing, prevention or elimination of a disorder, or side-effects. The amount needed to elicit the therapeutic response can be determined based on the age, health, size and sex of the subject. Optimal amounts can also be determined based on monitoring of the subject's response to treatment.

As used herein, the terms "treat," "treated," or "treating" mean both therapeutic treatment and prophylactic or preventative measures wherein the object is to prevent or slow down (lessen) an undesired physiological condition, disorder or disease, or obtain beneficial or desired clinical results. For purposes of this invention, beneficial or desired clinical results include, but are not limited to, alleviation of symptoms; diminishment of extent of condition, disorder or disease; stabilized (i.e., not worsening) state of condition, disorder or disease; delay in onset or slowing of condition, disorder or disease progression; amelioration of the condition, disorder or disease state or remission (whether partial or total), whether detectable or undetectable; an amelioration of at least one measurable physical parameter, not necessarily discernible by the patient; or enhancement or improvement of condition, disorder or disease. Treatment includes eliciting a clinically significant response without excessive levels of side effects. Treatment also includes prolonging survival as compared to expected survival if not receiving treatment.

The compounds of the disclosure are identified herein by their chemical structure and/or chemical name. Where a compound is referred to by both a chemical structure and a chemical name, and that chemical structure and chemical name conflict, the chemical structure is determinative of the compound's identity.

At various places in the present specification, substituents of compounds may be disclosed in groups or in ranges. It is specifically intended that the invention include each and every individual subcombination of the members of such groups and ranges. For example, the term "C₁₋₆alkyl" is specifically intended to individually disclose methyl, ethyl, propyl, C₄alkyl, C₅alkyl, and C₆alkyl, linear and/or branched.

For compounds in which a variable appears more than once, each variable can be a different moiety selected from the Markush group defining the variable. For example, where a structure is described having two R groups that are simultaneously present on the same compound, the two R groups can represent different moieties selected from the Markush groups defined for R. In another example, when an optionally multiple substituent is designated in the form, for example, 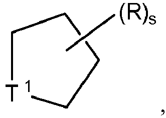 then it is understood that substituent R can occur "s" number of times on the ring, and R can be a different moiety at each occurrence. Further, in the above example, where the variable T¹ is defined to include hydrogens, such as when T¹ is CH₂, NH, etc., any H can be replaced with a substituent.

It is further appreciated that certain features of the disclosure, which are, for clarity, described in the context of separate embodiments, can also be provided in combination in a single embodiment. Conversely, various features of the disclosure which are, for brevity, described in the context of a single embodiment, can also be provided separately or in any suitable sub-combination.

It is understood that the present disclosure encompasses the use, where applicable, of stereoisomers, diastereomers and optical stereoisomers of the compounds of the disclosure, as well as mixtures thereof. Additionally, it is understood that stereoisomers, diastereomers, and optical stereoisomers of the compounds of the disclosure, and mixtures thereof, are within the scope of the disclosure. By way of non-limiting example, the mixture may be a racemate or the mixture may comprise unequal proportions of one particular stereoisomer over the other. Additionally, the compounds can be provided as a substantially pure stereoisomers, diastereomers and optical stereoisomers (such as epimers).

The compounds described herein can be asymmetric (e.g., having one or more stereocenters). All stereoisomers, such as enantiomers and diastereomers, are intended to be included within the scope of the disclosure unless otherwise indicated. Compounds that contain asymmetrically substituted carbon atoms can be isolated in optically active or racemic forms. Methods of preparation of optically active forms from optically active starting materials are known in the art, such as by resolution of racemic mixtures or by stereoselective synthesis. Many geometric isomers of olefins, C=N double bonds, and the like can also be present in the compounds described herein, and all such stable isomers are contemplated in the present disclosure. *Cis* and *trans* geometric isomers of the compounds are also included within the scope of the disclosure and can be isolated as a mixture of isomers or as separated isomeric forms. Where a compound capable of stereoisomerism or geometric isomerism is designated in its structure or name without reference to specific R/S or cis/trans configurations, it is intended that all such isomers are contemplated.

Resolution of racemic mixtures of compounds can be carried out by any of numerous methods known in the art, including, for example, fractional recrystallizaion using a chiral resolving acid which is an optically active, salt-forming organic acid. Suitable resolving agents for fractional recrystallization methods include, but are not limited to, optically active acids, such as the D and L forms of tartaric acid, diacetyltartaric acid, dibenzoyltartaric acid, mandelic acid, malic acid, lactic acid, and the various optically active camphorsulfonic acids such as β-camphorsulfonic acid. Other resolving agents suitable for fractional crystallization methods include, but are not limited to, stereoisomerically pure forms of α-methylbenzylamine (e.g., *S* and R forms, or diastereomerically pure forms), 2-phenylglycinol, norephedrine, ephedrine, N-methylephedrine, cyclohexylethylamine, 1,2-diaminocyclohexane, and the like. Resolution of racemic mixtures can also be carried out by elution on a column packed with an optically active resolving agent (e.g., dinitrobenzoylphenylglycine). Suitable elution solvent compositions can be determined by one skilled in the art.

Compounds may also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Examples of prototropic tautomers include, but are not limited to, ketone-enol pairs, amide-imidic acid pairs, lactam-lactim pairs, amide-imidic acid pairs, enamine-imine pairs, and annular forms where a proton can occupy two or more positions of a heterocyclic system including, but not limited to, 1H- and 3H-imidazole, 1H-, 2H- and 4H-1,2,4-triazole, 1H- and 2H- isoindole, and 1H- and 2H-pyrazole. Tautomeric forms can be in equilibrium or sterically locked into one form by appropriate substitution.

Compounds also include hydrates and solvates, as well as anhydrous and non-solvated forms.

Compounds can also include all isotopes of atoms occurring in the intermediates or final compounds. Isotopes include those atoms having the same atomic number but different mass numbers. For example, isotopes of hydrogen include tritium and deuterium.

As described herein, the compounds, or pharmaceutically acceptable salts thereof, may be substantially isolated. Partial separation can include, for example, a composition enriched in the compound of the disclosure. Substantial separation can include compositions containing at least about 50%, at least about 60%, at least about 70%, at least about 80%, at least about 90%, at least about 95%, at least about 97%, or at least about 99% by weight of the compound of the disclosure, or pharmaceutically acceptable salt thereof. Methods for isolating compounds and their salts are routine in the art.

Although the disclosed compounds are suitable, other functional groups can be incorporated into the compound with an expectation of similar results. In particular, thioamides and thioesters are anticipated to have very similar properties. The distance between aromatic rings can impact the geometrical pattern of the compound and this distance can be altered by incorporating aliphatic chains of varying length, which can be optionally substituted or can comprise an amino acid, a dicarboxylic acid or a diamine. The distance between and the relative orientation of monomers within the compounds can also be altered by replacing the amide bond with a surrogate having additional atoms. Thus, replacing a carbonyl group with a dicarbonyl alters the distance between the monomers and the propensity of dicarbonyl unit to adopt an anti arrangement of the two carbonyl moiety and alter the periodicity of the compound. Pyromellitic anhydride represents still another alternative to simple amide linkages which can alter the conformation and physical properties of the compound. Modern methods of solid phase organic chemistry (E. Atherton and R. C. Sheppard, Solid Phase Peptide Synthesis A Practical Approach IRL Press Oxford 1989) now allow the synthesis of homodisperse compounds with molecular weights approaching 5,000 Daltons. Other substitution patterns are equally effective.

The compounds described herein also include derivatives referred to as prodrugs, which can be prepared by modifying functional groups present in the compounds in such a way that the modifications are cleaved, either in routine manipulation or *in vivo,* to the parent compounds. Examples of prodrugs include compounds as described herein that contain one or more molecular moieties appended to a hydroxyl, amino, sulfhydryl, or carboxyl group of the compound, and that when administered to a patient, cleaves *in vivo* to form the free hydroxyl, amino, sulfhydryl, or carboxyl group, respectively. Examples of prodrugs include, but are not limited to, acetate, formate and benzoate derivatives of alcohol and amine functional groups in the compounds described herein. Preparation and use of prodrugs is discussed in T. Higuchi et al., "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

Compounds containing an amine function can also form N-oxides. A reference herein to a compound that contains an amine function also includes the N-oxide. Where a compound contains several amine functions, one or more than one nitrogen atom can be oxidized to form an N-oxide. Examples of N-oxides include N-oxides of a tertiary amine or a nitrogen atom of a nitrogen-containing heterocycle. N-Oxides can be formed by treatment of the corresponding amine with an oxidizing agent such as hydrogen peroxide or a per-acid (e.g., a peroxycarboxylic acid) (see, Advanced Organic Chemistry, by Jerry March, 4th Edition, Wiley Interscience).

The present disclosure provides compounds of Formula V: 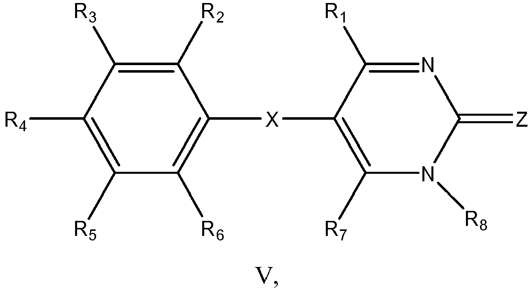
or a pharmaceutically acceptable salt thereof, wherein each of R₁, R₂, R₃, R₄, R₅, R₆, and R₇ are, independently, a suitable substituent; or wherein each of R₁, R₂, R₃, R₄, R₅, R₆, and R₇ are, independently, a hydrogen, alkoxy, alkyl, alkenyl, alkynyl, aryl, aryloxy, benzyl, cycloalkyl, halogen, heteroaryl, heterocycloalkyl, -CN, -OH, -NO₂, -CF₃, -CO₂H, -CO₂alkyl, or -NH₂;
R₈ is an alkyl or hydrogen;
X is O, S, NH, or N-akyl; and
Z is O or S.

As described herein, R₈ may be alkyl, such as methyl. As described herein, R₈ may be hydrogen.

As described herein, X may be oxygen.

As described herein, Z may be oxygen.

As described herein, at least one of R₂-R₆ may be alkyl, such as methyl. As described herein, at least one of R₂-R₆ may be halogen, such as chloro. As described herein, at least one of R₂-R₆ may be -CN. As described herein, at least one of R₂-R₆ may be -OH. As described herein, at least one of R₂-R₆ may be -NO₂. As described herein, at least one of R₂-R₆ may be -CF₃. As described herein, at least one of R₂-R₆ may be -CO₂H. As described herein, at least one of R₂-R₆ may be -NH₂. As described herein, at least one of R₂-R₆ may be -alkoxy.

As described herein, R₂ may be alkyl, such as methyl and each of R₁, and R₃-R₈ may be hydrogen, and X and Z may be O. As described herein, R₂ may be a halogen, such as chloro, and each of R₁, and R₃-R₈ may be hydrogen, and X and Z may be O. As described herein, R₃ may be alkyl, such as methyl, and each of R₁, R₂ and R₄-R₈ may be hydrogen, and X and Z may be O.

As described herein, R₃ may be a halogen, such as chloro, and each of R₁, R₂, and R₄-R₈ may be hydrogen, and X and Z may be O.

As described herein, R₄ may be alkyl, such as methyl, and each of R₁- R₃ and R₅-R₈ may be hydrogen, and X and Z may be O.

As described herein, R₄ may be a halogen, such as chloro, and each of R₁-R₃, and R₅-R₈ may be hydrogen, and X and Z may be O.

As described herein, R₅ may be -CF₃, and each of R₁-R₄ and R₆-R₈ may be hydrogen, and X and Z may be O. As described herein, R₅ may be -NH₂, and each of R₁-R₄ and R₆-R₈ may be hydrogen, and X and Z may be O.

As described herein, R₆ may be -CF₃, and each of R₁-R₅ and R₇-R₈ may be hydrogen, and X and Z may be O. As described herein, R₆ may be -NH₂ and each of R₁- R₅ and R₇-R₈ may be hydrogen, and X and Z may be O.

The present disclosure also provides compounds of Formula I 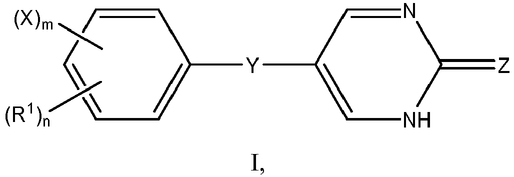 or a pharmaceutically acceptable salt thereof, wherein:
R¹ is an alkyl group;
X is a halogen;
Y is O, S, or NH;
Z is O or S;
n is an integer from 0 to 5 and m is 0 or 1, wherein m + n is less than or equal to 5.

As described herein, the alkyl group may be methyl and n may be 1.

As described herein, the halogen may be chlorine and m may be 1.

As described herein, Y may be O.

As described herein, Z may be O.

As described herein, R¹ may be methyl, Y may be O, Z may be O, n may be 1, and m may be 0. As described herein, R¹ may be in the meta position.

As described herein, X may be chlorine, Y may be O, Z may be O, n may be 0, and m may be 1. As described herein, X may be in the meta position.

The present disclosure also provides compounds of Formula II: 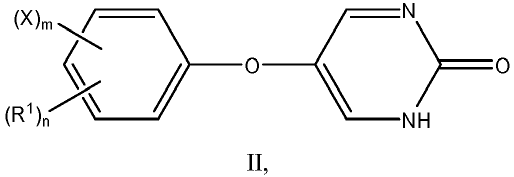 or a pharmaceutically acceptable salt thereof, wherein:
R¹ is an alkyl group;
X is a halogen; and
n is an integer from 0 to 5 and m is 0 or 1, wherein m + n is less than or equal to 5.

As described herein, the alkyl group may be methyl and n may be 1.

As described herein, the halogen may be chlorine and m may be 1.

As described herein, R¹ may be methyl, n may be 1, and m may be 0. As described herein, R¹ may be in the meta position.

As described herein, X may be chlorine, n may be 0, and m may be 1. As described herein, X may be in the meta position.

The present disclosure also provides compounds of Formula III: 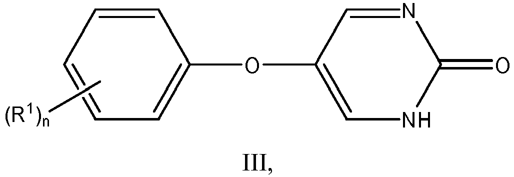 or a pharmaceutically acceptable salt thereof, wherein:
R¹ is an alkyl group; and
n is an integer from 0 to 5.

As described herein, R¹ may be methyl, n may be 1. As described herein, R¹ may be in the meta position.

The present disclosure also provides compounds of Formula IV: 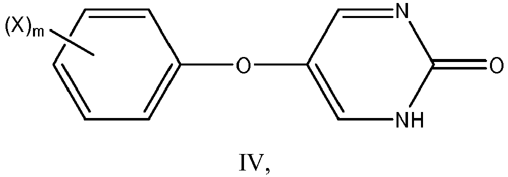 or a pharmaceutically acceptable salt thereof, wherein:
X is a halogen; and
m is an integer from 0 to 1.

As described herein, X may be chloro and m may be 1. As described herein, X may be in the meta position.

Illustrative examples of compounds that are encompassed by Formulas I-IV and that are useful in the methods described herein include, but are not limited to: 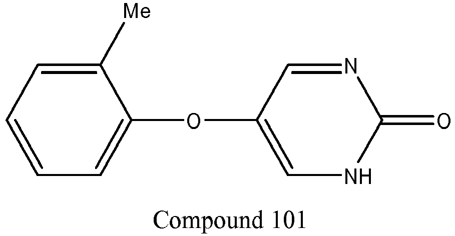 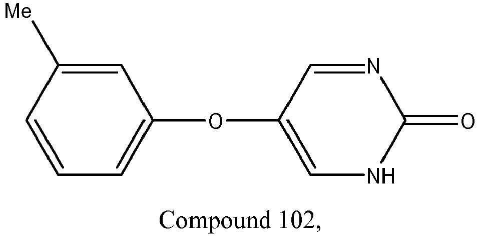 which is also known as Tolimidone, CP-26154, and 2(1H)-Pyrimidinone, 5-(3-methylphenoxy).

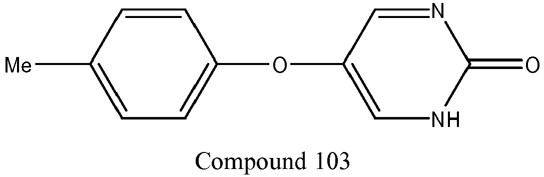

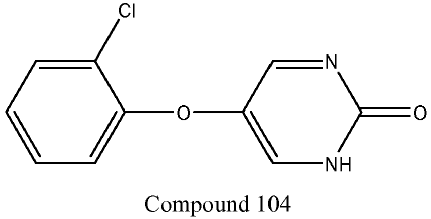

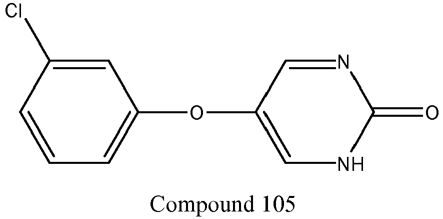

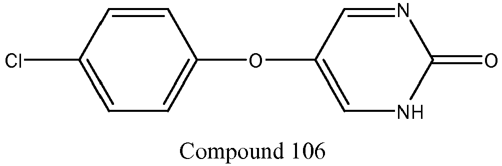

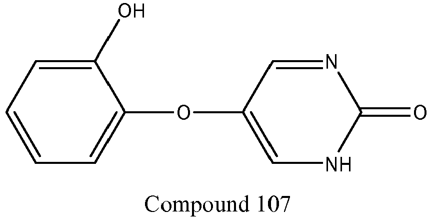

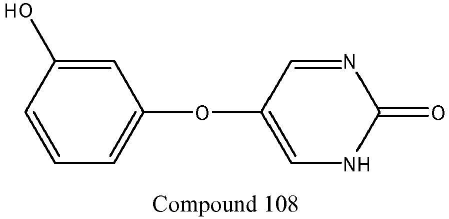

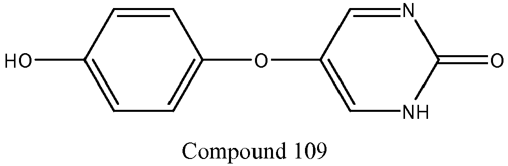

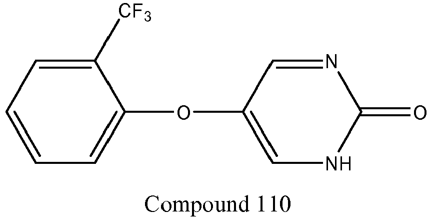

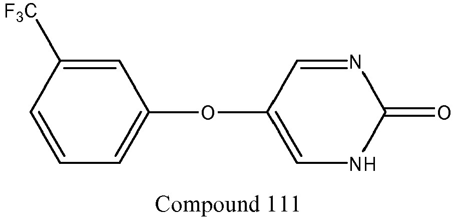

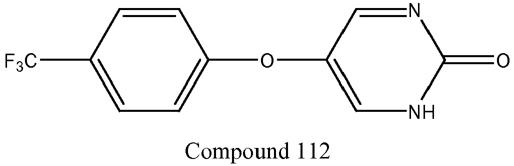

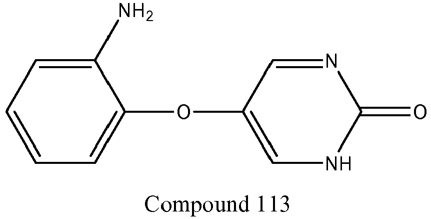

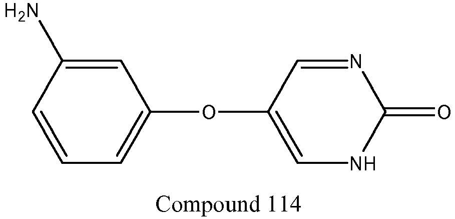

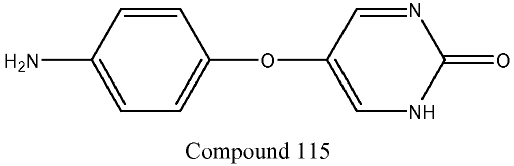

The compounds described herein can be synthesized by organic chemistry techniques known to those of ordinary skill in the art, for example as described in U.S. patent number 3,922,345.

The compounds of the disclosure are present in compositions comprising insulin and are administered to a mammal therewith. Suitable insulins include, but are not limited to, injectable insulin, transdermal insulin, inhaled insulin, or any combination thereof. As an alternative to insulin, an insulin derivative, secretagogue, sensitizer or mimetic may be used. Insulin secretagogues for use in combination with the compounds of the disclosure include, but are not limited to, forskolin, dibutryl cAMP, and isobutylmethylxanthine (IBMX).

There are four types of insulin that are distinguished by their onset and duration for action. Rapid-acting insulins (e.g. Humalog) have a rapid onset (within 15 minutes) and a duration of action of up to 5 hours and cover needs for meals ingested at the time of injection. Short-acting insulins (e.g. Humulin) also have a rapid onset, cover needs for meals ingested within an hour of administration and have a duration of action of up to 8 hours. Intermediate acting insulins (e.g. NPH) have a delayed onset (1-2 hours) and a duration of action of up to 24 hours. Long-acting insulins (e.g. Lantus, Levemir) have a delayed onset with durations of action up to 36 hours after administration. These insulins are typically administered in various combinations to modulate blood glucose levels.

The compounds described herein can be administered in any conventional manner by any route where they are active. Administration can be systemic, topical, or oral. For example, administration can be, but is not limited to, parenteral, subcutaneous, intravenous, intramuscular, intraperitoneal, transdermal, oral, buccal, sublingual, or ocular routes, or intravaginally, by inhalation, by depot injections, or by implants. The mode of administration can depend on the pathogen or microbe to be targeted. The selection of the specific route of administration can be selected or adjusted by the clinician according to methods known to the clinician to obtain the desired clinical response.

As described herein, it may be desirable to administer one or more compounds, or a pharmaceutically acceptable salt thereof, locally to an area in need of treatment. This may be achieved, for example, and not by way of limitation, by local infusion during surgery, topical application, e.g., in conjunction with a wound dressing after surgery, by injection, by means of a catheter, by means of a suppository, or by means of an implant, wherein the implant is of a porous, non-porous, or gelatinous material, including membranes, such as sialastic membranes, or fibers.

As described herein, the compounds of the disclosure can be used in combination therapy with insulin and at least one other therapeutic agent. The compound of the disclosure, insulin, and the therapeutic agent can act additively or synergistically. As described herein, a composition comprising a compound of the disclosure may be administered concurrently or serially with the administration of insulin and another therapeutic agent, which can be part of the same composition as the compound of the disclosure or a different composition. As described herein, a composition comprising a compound of the disclosure may be administered prior or subsequent to administration of insulin and another therapeutic agent. As many of the disorders for which the compounds of the disclosure are useful in treating are chronic disorders, combination therapy may involve alternating between administering a composition comprising a compound of the disclosure, insulin, and a composition comprising another therapeutic agent, e.g., to minimize the toxicity associated with a particular therapeutic agent. The duration of administration of each drug or therapeutic agent can be, e.g., one month, three months, six months, or a year. As described herein, when a composition of the disclosure is administered concurrently with insulin and another therapeutic agent that potentially produces adverse side effects including, but not limited to, toxicity, the therapeutic agent can advantageously be administered at a dose that falls below the threshold at which the adverse side is elicited. As described herein, the compounds can be administered in combination with insulin and another diabetes drug, blood pressure drug, and/or cholesterol drug.

The present compositions can be administered together with a statin. Statins for use in combination with the compounds of the disclosure and insulin include, but are not limited to, atorvastatin, pravastatin, fluvastatin, lovastatin, simvastatin, and cerivastatin.

The present compositions can also be administered together with a PPAR agonist, for example a thiazolidinedione or a fibrate. Thiazolidinediones for use in combination with the compounds of the disclosure and insulin include, but are not limited to, pioglitazone, ciglitazone, 5-((4-(2-(methyl-2-pyridinylamino)ethoxy)phenyl)methyl)-2,4-thiazolidinedione, troglitazone, WAY-120,744, englitazone, AD 5075, darglitazone, and rosiglitazone. Fibrates for use in combination with the compounds of the disclosure and insulin include but are not limited to gemfibrozil, fenofibrate, clofibrate, or ciprofibrate. As mentioned previously, a therapeutically effective amount of a fibrate or thiazolidinedione often has toxic side effects. Accordingly, As described herein , when a composition of the discosure is administered in combination with insulin and a PPAR agonist, the dosage of the PPAR agonist is below that which is accompanied by toxic side effects.

The present compositions can also be administered together with a bile-acid-binding resin. Bile-acid-binding resins for use in combination with the compounds of the disclosure and insulin include, but are not limited to, cholestyramine and colestipol hydrochloride.

The present compositions can also be administered together with niacin or nicotinic acid.

The present compositions can also be administered together with a RXR agonist. RXR agonists for use in combination with the compounds of the disclosure and insulin include, but are not limited to, LG 100268, LGD 1069, 9-cis retinoic acid, 2-(1-(3,5,5,8,8-pentamethyl-5,6,7,8-tetrahydro-2-naphthyl)-cyclopropyl)-pyridine-5-carboxylic acid, or 4-((3,5,5,8,8-pentamethyl-5,6,7,8-tetrahydro-2-naphthyl)2-carbonyl)-benzoic acid.

The present compositions can also be administered together with an anti-obesity drug. Anti-obesity drugs for use in combination with the compounds of the disclosure and insulin include, but are not limited to, β-adrenergic receptor agonists, such as β-3 receptor agonists, sibutramine, bupropion, fluoxetine, and phentermine.

The present compositions can also be administered together with a hormone. Hormones for use in combination with the compounds of the disclosure and insulin include, but are not limited to, thyroid hormone and estrogen.

The present compositions can also be administered together with a tyrophostine or an analog thereof. Tyrophostines for use in combination with the compounds of the disclosure and insulin include, but are not limited to, tryophostine 51.

The present compositions can also be administered together with sulfonylurea-based drugs. Sulfonylurea-based drugs for use in combination with the compounds of the disclosure and insulin include, but are not limited to, glisoxepid, glyburide, acetohexamide, chlorpropamide, glibornuride, tolbutamide, tolazamide, glipizide, gliclazide, gliquidone, glyhexamide, phenbutamide, and tolcyclamide.

The present compositions can also be administered together with a biguanide. Biguanides for use in combination with the compounds of the disclosure and insulin include, but are not limited to, metformin, phenformin, and buformin.

The present compositions can also be administered together with an α-glucosidase inhibitor. α-glucosidase inhibitors for use in combination with the compounds of the disclosure and insulin include, but are not limited to, acarbose and miglitol.

The present compositions can also be administered together with an apo A-I agonist. As described herein, the apo A-I agonist may be the Milano form of apo A-I (apo A-IM). As described herein, the apo A-IM for administration in conjunction with the compounds of the disclosure and insulin may be produced by the method of U.S. Pat. No. 5,721,114 to Abrahamsen. As described herein, the apo A-I agonist may be a peptide agonist. As described herein, the apo A-I peptide agonist for administration in conjunction with the compounds of the disclosure and insulin may be a peptide of U.S. Pat. No. 6,004,925 or 6,037,323.

The present compositions can also be administered together with apolipoprotein E (apo E). As described herein, the apoE for administration in conjunction with the compounds of the disclosure and insulin may be produced by the method of U.S. Pat. No. 5,834,596.

As described herein, the present compositions can be administered together with an HDL-raising drug; an HDL enhancer; or a regulator of the apolipoprotein A-I, apolipoprotein A-IV and/or apolipoprotein genes.

The present compositions can be administered together with a known cardiovascular drug. Cardiovascular drugs for use in combination with the compounds of the disclosure and insulin to prevent or treat cardiovascular diseases include, but are not limited to, peripheral anti-adrenergic drugs, centrally acting antihypertensive drugs (e.g., methyldopa, methyldopa HCl), antihypertensive direct vasodilators (e.g., diazoxide, hydralazine HCl), drugs affecting reninangiotensin system, peripheral vasodilators, phentolamine, antianginal drugs, cardiac glycosides, inodilators (e.g., amrinone, milrinone, enoximone, fenoximone, imazodan, sulmazole), antidysrhythmic drugs, calcium entry blockers, ranitine, bosentan, and rezulin.

The present compositions can be administered together with treatment with irradiation or one or more chemotherapeutic agents. For irradiation treatment, the irradiation can be gamma rays or X-rays. For a general overview of radiation therapy, see Hellman, Chapter 12: Principles of Radiation Therapy Cancer, in: Principles and Practice of Oncology, DeVita et al., eds., 2nd. Ed., J.B. Lippencott Company, Philadelphia. Useful chemotherapeutic agents include methotrexate, taxol, mercaptopurine, thioguanine, hydroxyurea, cytarabine, cyclophosphamide, ifosfamide, nitrosoureas, cisplatin, carboplatin, mitomycin, dacarbazine, procarbizine, etoposides, campathecins, bleomycin, doxorubicin, idarubicin, daunorubicin, dactinomycin, plicamycin, mitoxantrone, asparaginase, vinblastine, vincristine, vinorelbine, paclitaxel, and docetaxel. As described herein, a composition of the invention further comprises one or more chemotherapeutic agents and/or may be administered concurrently with radiation therapy. As described herein, chemotherapy or radiation therapy may be administered prior or subsequent to administration of a present composition, such as at least an hour, five hours, 12 hours, a day, a week, a month, or several months (e.g., up to three months), subsequent to administration of a composition of the disclosure.

The amount of compound, insulin, and other therapeutic agent to be administered is that amount which is therapeutically effective. The dosage to be administered will depend on the characteristics of the subject being treated, e.g., the particular animal treated, age, weight, health, types of concurrent treatment, if any, and frequency of treatments, and can be easily determined by one of skill in the art (e.g., by the clinician). The selection of the specific dose regimen can be selected or adjusted or titrated by the clinician according to methods known to the clinician to obtain the desired clinical response.

The amount of a compound described herein, insulin, and other therapeutic agent that will be effective in the treatment and/or prevention of a particular disease, condition, or disorder will depend on the nature and extent of the disease, condition, or disorder, and can be determined by standard clinical techniques. In addition, *in vitro* or *in vivo* assays may optionally be employed to help identify optimal dosage ranges. The precise dose to be employed in the compositions will also depend on the route of administration, and the seriousness of the disorder, and should be decided according to the judgment of the practitioner and each patient's circumstances. However, a suitable dosage range for oral administration is, generally, from about 0.001 milligram to about 200 milligrams per kilogram body weight, from about 0.01 milligram to about 100 milligrams per kilogram body weight, from about 0.01 milligram to about 70 milligrams per kilogram body weight, from about 0.1 milligram to about 50 milligrams per kilogram body weight, from 0.5 milligram to about 20 milligrams per kilogram body weight, or from about 1 milligram to about 10 milligrams per kilogram body weight. As described herein, the oral dose may be about 5 milligrams per kilogram body weight.

As described herein, suitable dosage ranges for intravenous (i.v.) administration may be from about 0.01 mg to about 500 mg per kg body weight, from about 0.1 mg to about 100 mg per kg body weight, from about 1 mg to about 50 mg per kg body weight, or from about 10 mg to about 35 mg per kg body weight. Suitable dosage ranges for other modes of administration can be calculated based on the forgoing dosages as known by those skilled in the art. For example, recommended dosages for intradermal, intramuscular, intraperitoneal, subcutaneous, epidural, sublingual, intracerebral, intravaginal, transdermal administration or administration by inhalation are in the range of from about 0.001 mg to about 200 mg per kg of body weight, from about 0.01 mg to about 100 mg per kg of body weight, from about 0.1 mg to about 50 mg per kg of body weight, or from about 1 mg to about 20 mg per kg of body weight. Effective doses may be extrapolated from dose-response curves derived from *in vitro* or animal model test systems. Such animal models and systems are well known in the art.

The compounds described herein can be formulated for parenteral administration by injection, such as by bolus injection or continuous infusion. The compounds can be administered by continuous infusion subcutaneously over a period of about 15 minutes to about 24 hours. Formulations for injection can be presented in unit dosage form, such as in ampoules or in multidose containers, with an added preservative. The compositions can take such forms as suspensions, solutions or emulsions in oily or aqueous vehicles, and can contain formulatory agents such as suspending, stabilizing and/or dispersing agents. As described herein, the injectable may be in the form of short-acting, depot, or implant and pellet forms injected subcutaneously or intramuscularly. As described herein, the parenteral dosage form may be the form of a solution, suspension, emulsion, or dry powder.

For oral administration, the compounds described herein can be formulated by combining the compounds with pharmaceutically acceptable carriers well known in the art. Such carriers enable the compounds to be formulated as tablets, pills, dragees, capsules, emulsions, liquids, gels, syrups, caches, pellets, powders, granules, slurries, lozenges, aqueous or oily suspensions, and the like, for oral ingestion by a patient to be treated. Pharmaceutical preparations for oral use can be obtained by, for example, adding a solid excipient, optionally grinding the resulting mixture, and processing the mixture of granules, after adding suitable auxiliaries, if desired, to obtain tablets or dragee cores. Suitable excipients include, but are not limited to, fillers such as sugars, including, but not limited to, lactose, sucrose, mannitol, and sorbitol; cellulose preparations such as, but not limited to, maize starch, wheat starch, rice starch, potato starch, gelatin, gum tragacanth, methyl cellulose, hydroxypropylmethyl-cellulose, sodium carboxymethylcellulose, and polyvinylpyrrolidone (PVP). If desired, disintegrating agents can be added, such as, but not limited to, the cross-linked polyvinyl pyrrolidone, agar, or alginic acid or a salt thereof such as sodium alginate.

Orally administered compositions can contain one or more optional agents, for example, sweetening agents such as fructose, aspartame or saccharin; flavoring agents such as peppermint, oil of wintergreen, or cherry; coloring agents; and preserving agents, to provide a pharmaceutically palatable preparation. Moreover, where in tablet or pill form, the compositions may be coated to delay disintegration and absorption in the gastrointestinal tract thereby providing a sustained action over an extended period of time. Selectively permeable membranes surrounding an osmotically active driving compound are also suitable for orally administered compounds. Oral compositions can include standard vehicles such as mannitol, lactose, starch, magnesium stearate, sodium saccharine, cellulose, magnesium carbonate, etc. Such vehicles are suitably of pharmaceutical grade.

Dragee cores can be provided with suitable coatings. For this purpose, concentrated sugar solutions can be used, which can optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or dragee coatings for identification or to characterize different combinations of active compound doses.

Pharmaceutical preparations which can be used orally include, but are not limited to, push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added.

For buccal administration, the compositions can take the form of, such as, tablets or lozenges formulated in a conventional manner.

For administration by inhalation, the compounds described herein can be delivered in the form of an aerosol spray presentation from pressurized packs or a nebulizer, with the use of a suitable propellant, such as dichlorodifluoromethane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of, such as gelatin for use in an inhaler or insufflator can be formulated containing a powder mix of the compound and a suitable powder base such as lactose or starch.

The compounds described herein can also be formulated in rectal compositions such as suppositories or retention enemas, such as containing conventional suppository bases such as cocoa butter or other glycerides. The compounds described herein can also be formulated in vaginal compositions such as vaginal creams, suppositories, pessaries, vaginal rings, and intrauterine devices.

In transdermal administration, the compounds can be applied to a plaster, or can be applied by transdermal, therapeutic systems that are consequently supplied to the organism. As described herein, the compounds may be present in creams, solutions, powders, fluid emulsions, fluid suspensions, semi-solids, ointments, pastes, gels, jellies, and foams, or in patches containing any of the same.

The compounds described herein can also be formulated as a depot preparation. Such long acting formulations can be administered by implantation (for example subcutaneously or intramuscularly) or by intramuscular injection. Depot injections can be administered at about 1 to about 6 months or longer intervals. Thus, for example, the compounds can be formulated with suitable polymeric or hydrophobic materials (for example as an emulsion in an acceptable oil) or ion exchange resins, or as sparingly soluble derivatives, for example, as a sparingly soluble salt.

As described herein, the compounds can be delivered in a controlled release system. As described herein, a pump may be used (see Langer, supra; Sefton, CRC Crit. Ref. Biomed. Eng., 1987, 14, 201; Buchwald et al., Surgery, 1980, 88, 507 Saudek et al., N. Engl. J. Med., 1989, 321, 574). As described herein, polymeric materials can be used (see Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger et al., J. Macromol. Sci. Rev. Macromol. Chem., 1983, 23, 61; see, also Levy et al., Science, 1985, 228, 190; During et al., Ann. Neurol., 1989, 25, 351; Howard et al., J. Neurosurg., 1989, 71, 105). As described herein, a controlled-release system can be placed in proximity of the target of the compounds described herein, such as the liver, thus requiring only a fraction of the systemic dose (see, e.g., Goodson, in Medical Applications of Controlled Release, supra, vol. 2, pp. 115-138 (1984)). Other controlled-release systems discussed in the review by Langer, Science, 1990, 249, 1527-1533) may be used.

It is also known in the art that the compounds can be contained in such formulations with pharmaceutically acceptable diluents, fillers, disintegrants, binders, lubricants, surfactants, hydrophobic vehicles, water soluble vehicles, emulsifiers, buffers, humectants, moisturizers, solubilizers, preservatives and the like. The pharmaceutical compositions can also comprise suitable solid or gel phase carriers or excipients. Examples of such carriers or excipients include, but are not limited to, calcium carbonate, calcium phosphate, various sugars, starches, cellulose derivatives, gelatin, and polymers such as polyethylene glycols. As described herein, the compounds described herein can be used with agents including, but not limited to, topical analgesics (e.g., lidocaine), barrier devices (e.g., GelClair), or rinses (e.g., Caphosol).

As described herein, the compounds described herein can be delivered in a vesicle, in particular a liposome (see, Langer, Science, 1990, 249, 1527-1533; Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; see generally ibid.).

Suitable compositions include, but are not limited to, oral non-absorbed compositions. Suitable compositions also include, but are not limited to saline, water, cyclodextrin solutions, and buffered solutions of pH 3-9.

The compounds described herein, or pharmaceutically acceptable salts thereof, can be formulated with numerous excipients including, but not limited to, purified water, propylene glycol, PEG 400, glycerin, DMA, ethanol, benzyl alcohol, citric acid/sodium citrate (pH3), citric acid/sodium citrate (pH5), tris(hydroxymethyl)amino methane HCl (pH7.0), 0.9% saline, and 1.2% saline, and any combination thereof. As described herein, excipient may be chosen from propylene glycol, purified water, and glycerin.

As described herein, the formulation can be lyophilized to a solid and reconstituted with, for example, water prior to use.

When administered to a mammal (e.g., to an animal for veterinary use or to a human for clinical use) the compounds can be administered in isolated form.

When administered to a human, the compounds can be sterile. Water is a suitable carrier when the compound is administered intravenously. Saline solutions and aqueous dextrose and glycerol solutions can also be employed as liquid carriers, particularly for injectable solutions. Suitable pharmaceutical carriers also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The present compositions, if desired, can also contain minor amounts of wetting or emulsifying agents, or pH buffering agents.

The compositions described herein can take the form of a solution, suspension, emulsion, tablet, pill, pellet, capsule, capsule containing a liquid, powder, sustained-release formulation, suppository, aerosol, spray, or any other form suitable for use. Examples of suitable pharmaceutical carriers are described in Remington's Pharmaceutical Sciences, A.R. Gennaro (Editor) Mack Publishing Co.

As described herein, the compounds may be formulated in accordance with routine procedures as a pharmaceutical composition adapted for administration to humans. Typically, compounds are solutions in sterile isotonic aqueous buffer. Where necessary, the compositions can also include a solubilizing agent. Compositions for intravenous administration may optionally include a local anesthetic such as lidocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the compound is to be administered by infusion, it can be dispensed, for example, with an infusion bottle containing sterile pharmaceutical grade water or saline. Where the compound is administered by injection, an ampoule of sterile water for injection or saline can be provided so that the ingredients may be mixed prior to administration.

The pharmaceutical compositions can be in unit dosage form. In such form, the composition can be divided into unit doses containing appropriate quantities of the active component. The unit dosage form can be a packaged preparation, the package containing discrete quantities of the preparations, for example, packeted tablets, capsules, and powders in vials or ampules. The unit dosage form can also be a capsule, cachet, or tablet itself, or it can be the appropriate number of any of these packaged forms.

As described herein, a composition of the present invention may be in the form of a liquid wherein the active agent (i.e., one of the facially amphiphilic polymers or oligomers disclosed herein) is present in solution, in suspension, as an emulsion, or as a solution/suspension. As described herein, the liquid composition may be in the form of a gel. As described herein, the liquid composition may be aqueous. As described herein, the composition may be in the form of an ointment.

Suitable preservatives include, but are not limited to, mercury-containing substances such as phenylmercuric salts (e.g., phenylmercuric acetate, borate and nitrate) and thimerosal; stabilized chlorine dioxide; quaternary ammonium compounds such as benzalkonium chloride, cetyltrimethylammonium bromide and cetylpyridinium chloride; imidazolidinyl urea; parabens such as methylparaben, ethylparaben, propylparaben and butylparaben, and salts thereof; phenoxyethanol; chlorophenoxyethanol; phenoxypropanol; chlorobutanol; chlorocresol; phenylethyl alcohol; disodium EDTA; and sorbic acid and salts thereof.

Optionally one or more stabilizers can be included in the compositions to enhance chemical stability where required. Suitable stabilizers include, but are not limited to, chelating agents or complexing agents, such as, for example, the calcium complexing agent ethylene diamine tetraacetic acid (EDTA). For example, an appropriate amount of EDTA or a salt thereof, e.g., the disodium salt, can be included in the composition to complex excess calcium ions and prevent gel formation during storage. EDTA or a salt thereof can suitably be included in an amount of about 0.01% to about 0.5%. Compositions containing a preservative other than EDTA, the EDTA or a salt thereof, more particularly disodium EDTA, can be present in an amount of about 0.025% to about 0.1% by weight.

One or more antioxidants can also be included in the compositions. Suitable antioxidants include, but are not limited to, ascorbic acid, sodium metabisulfite, sodium bisulfite, acetylcysteine, polyquaternium-1, benzalkonium chloride, thimerosal, chlorobutanol, methyl paraben, propyl paraben, phenylethyl alcohol, edetate disodium, sorbic acid, or other agents know to those of skill in the art. Such preservatives are typically employed at a level of from about 0.001% to about 1.0% by weight.

As described herein, the compounds may be solubilized at least in part by an acceptable solubilizing agent. Certain acceptable nonionic surfactants, for example polysorbate 80, can useful as solubilizing agents, as can acceptable glycols, polyglycols, e.g., polyethylene glycol 400 (PEG-400), and glycol ethers.

Suitable solubilizing agents for solution and solution/suspension compositions are cyclodextrins. Suitable cyclodextrins can be chosen from α-cyclodextrin, β-cyclodextrin, γ-cyclodextrin, alkylcyclodextrins (e.g., methyl-β-cyclodextrin, dimethyl-β-cyclodextrin, diethyl-β-cyclodextrin), hydroxyalkylcyclodextrins (e.g., hydroxyethyl-β-cyclodextrin, hydroxypropyl-β-cyclodextrin), carboxy-alkylcyclodextrins (e.g., carboxymethyl-β-cyclodextrin), sulfoalkylether cyclodextrins (e.g., sulfobutylether-β-cyclodextrin), and the like. Applications of cyclodextrins have been reviewed in Rajewski et al., Journal of Pharmaceutical Sciences, 1996, 85, 1155-1159. An acceptable cyclodextrin can optionally be present in a composition at a concentration from about 1 to about 200 mg/ml, from about 5 to about 100 mg/ml, or from about 10 to about 50 mg/ml.

As described herein, the composition optionally contains a suspending agent. For example, where the composition is an aqueous suspension or solution/suspension, the composition can contain one or more polymers as suspending agents. Useful polymers include, but are not limited to, water-soluble polymers such as cellulosic polymers, for example, hydroxypropyl methylcellulose, and water-insoluble polymers such as cross-linked carboxyl-containing polymers. However, where compositions do not contain substantial amounts of solid particulate matter, whether of the anti-microbial polymer or oligomer active agent, an excipient, or both, as solid particulate matter, if present, can cause discomfort and/or irritation of a treated eye.

One or more acceptable pH adjusting agents and/or buffering agents can be included in the compositions, including acids such as acetic, boric, citric, lactic, phosphoric and hydrochloric acids; bases such as sodium hydroxide, sodium phosphate, sodium borate, sodium citrate, sodium acetate, sodium lactate and tris-hydroxymethylaminomethane; and buffers such as citrate/dextrose, sodium bicarbonate and ammonium chloride. Such acids, bases and buffers are included in an amount required to maintain pH of the composition in an acceptable range.

Optionally one or more acceptable surfactants, preferably nonionic surfactants, or co-solvents may be included in the compositions to enhance solubility of the components of the compositions or to impart physical stability, or for other purposes. Suitable nonionic surfactants include, but are not limited to, polyoxyethylene fatty acid glycerides and vegetable oils, e.g., polyoxyethylene (60) hydrogenated castor oil; and polyoxyethylene alkylethers and alkylphenyl ethers, e.g., octoxynol 10, octoxynol 40; polysorbate 20, 60 and 80; polyoxyethylene / polyoxypropylene surfactants (e.g., Pluronic® F-68, F84 and P-103); cyclodextrin; or other agents known to those of skill in the art. Typically, such co-solvents or surfactants are employed in the compositions at a level of from about 0.01% to about 2% by weight.

One or more lubricating agents can also be included optionally in the compositions to promote lacrimation or as a "dry eye" medication. Such agents include, but are not limited to, polyvinyl alcohol, methylcellulose, hydroxypropyl methylcellulose, polyvinylpyrrolidone, and the like. It will be understood that promotion of lacrimation is beneficial in the present invention only where lacrimation is naturally deficient, to restore a normal degree of secretion of lacrimal fluid. Where excessive lacrimation occurs, residence time of the composition in the eye can be reduced.

The present disclosure also provides pharmaceutical packs or kits comprising one or more containers filled with one or more compounds or compositions described herein. Optionally associated with such container(s) can be a notice in the form prescribed by a governmental agency regulating the manufacture, use or sale of pharmaceuticals or biological products, which notice reflects approval by the agency of manufacture, use or sale for human administration for treating a condition, disease, or disorder described herein. As described herein, the kit contains more than one compound or composition described herein. As described herein, the kit comprises a compound described herein and insulin, and optionally another therapeutic agent, in a single injectable dosage form, such as a single dose within an injectable device such as a syringe with a needle.

The present disclosure also provides methods of treating Type I diabetes and/or Type II diabetes in a mammal comprising administering to the mammal in need thereof an effective amount of insulin and an acceptable amount of one or more compounds described above, or a pharmaceutically acceptable salt thereof. As described herein, the mammal can be pre-diagnosed with a Type I or Type II diabetes or pre-diabetes prior to treatment. As described herein, no formal diagnosis may have been made; the mammal may be suspected of having Type I or Type II diabetes or pre-diabetes for which treatment is recognized as being desirable. As described herein, the methods can be used to treat complications of obesity and diabetes such as, for example, hypercholesterolemia, hypertension, coronary heart disease; diabetic neuropathy, diabetic retinopathy, erectile dysfunction, and kidney disease.

As described herein, the compounds described herein can be administered either concurrently or serially with insulin. Thus, insulin may be administered first, followed by a compound described herein of any one of Formulas I-V. As described herein, a compound described herein of any one of Formulas I-V may be administered first, followed by insulin. As described herein, insulin and a compound described herein of any one of Formulas I-V may be administered simultaneously. When administered simultaneously, the insulin and the compound described herein of any one of Formulas I-V can be present in separate pharmaceutical compositions or may be combined into a single pharmaceutical composition, such as any one of the many pharmaceutical compositions described herein. As described herein, the present administration regimen for insulin can be used for administration of both insulin and a compound of any one of Formulas I-V described herein.

As described herein, the effective amount of any one of the compounds of Formula I-V may be from about 0.1 mg/kg to about 100 mg/kg, from about 0.5 mg/kg to about 50 mg/kg, from about 1 mg/kg to about 25 mg/kg, or from about 5 mg/kg to about 20 mg/kg.

Dose-levels and frequency of insulin injections vary depending on type of diabetes (Type I or II), state of insulin receptor sensitivity, age, and blood glucose levels and are tailored for the individual patient. Starting doses for the insulins are as follows: Rapid acting insulins, such as Humalog, are administered at a starting dose of 0.5 U/kg/day; short-acting insulins, such as Humulin, are administered at a starting dose of 0.5 U/kg/day; intermediate acting insulins, such as NPH, and long-acting insulins, such as Lantus, are administered at a starting dose of 0.2 U/kg/day.

As described herein, rapid acting insulins, such as Humalog, or short-acting insulins, such as Humulin, may be administered (along with a compound of Formula I-V) at a starting dose from about 0.05 U/kg/day to about 5 U/kg/day, from about 0.1 U/kg/day to about 2.5 U/kg/day, or from about 0.3 U/kg/day to about 0.8 U/kg/day. As described herein, rapid acting insulins, such as Humalog, or short-acting insulins, such as Humulin, may be administered (along with a compound of Formula I-V) at a starting dose of about 0.5 U/kg/day.

As described herein, intermediate acting insulins, such as NPH, and long-acting insulins, such as Lantus, may be administered (along with a compound of Formula I-V) at a starting dose from about 0.01 U/kg/day to about 3 U/kg/day, from about 0.05 U/kg/day to about 0.6 U/kg/day, or from about 0.1 U/kg/day to about 0.3 U/kg/day. As described herein, intermediate acting insulins, such as NPH, and long-acting insulins, such as Lantus, may be administered (along with a compound of Formula I-V) at a starting dose of about 0.02 U/kg/day.

In order that the present disclosure may be more efficiently understood, examples are provided below. It should be understood that these examples are for illustrative purposes only and are not to be construed as limiting the claimed embodiments in any manner. Throughout these examples, molecular cloning reactions, and other standard recombinant DNA techniques, were carried out according to methods described in Maniatis et al., Molecular Cloning - A Laboratory Manual, 2nd ed., Cold Spring Harbor Press (1989), using commercially available reagents, except where otherwise noted.

### Examples

### Example 1: Db/db Study 1

In the present study, male DbDb Lep-deficient mice (Harlan Laboratories) approximately 16 weeks of age were used. The experimental design is shown in Table 1.

**Table 1. Experimental Design Study 1**

| **Treatment** | **Insulin (dose-level; units/kg)** | **Compound 102 (dose-level; mg/kg)** | **group size** | |
|---|---|---|---|---|
| Vehicle | 0 | 0 | 6 | Blood Glucose |
| Compound 102 | 0 | 100 | 6 | Blood Glucose |
| Insulin | 3 | 0 | 6 | Blood Glucose |
| Compound 102/Insulin | 3 | 100 | 6 | Blood Glucose |

*Procedure:* Mice were divided into 4 groups of 6 mice per group. Blood glucose levels were measured using Accu-Chek Aviva Glucometers (Roche Diagnostics). The times of blood glucose levels were measured up to 72 hours after administration. Glucometers were calibrated prior to each study. Blood (5 µL) was acquired from a tail snip and directly applied to a glucose test strip. Glucose levels are reported as mg/dL.

In study 1, animals were fasted for 4 hours prior to the administration of test articles and then continuously fasted through to the 8-hour time point. Animals were then provided food *ad libitum* until 4-hour prior to the 28-hour time point at which point they were fasted until the last blood glucose measurement time point.

Data are expressed as the average ± SEM. Data were analyzed by two-way ANOVA followed by post-hoc Bonferroni test. N=6/group.

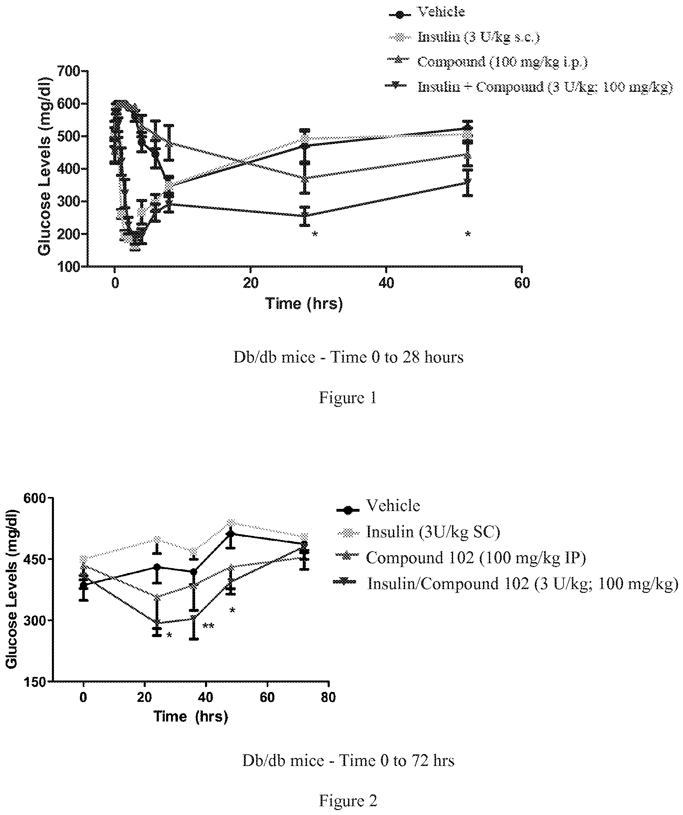

*Results:* In study 1, 16 week old db/db mice exhibited fasted blood glucose levels that averaged 482 mg/dL. Insulin reduced blood glucose levels that averaged 166.5 mg/dL three hours after administration. Blood glucose levels returned to baseline levels in the insulin-treated animals by 8 hours. Administration of Compound 102 did not significantly affect blood glucose levels at any time point after administration. However, co-administration of Compound 102 with insulin prolonged the insulin response with significant blood glucose lowering observed until at least 52 hours after administration. Historical data from this laboratory shows that 16 week-old db/db mice have insulin levels less than or equal to 1 ng/mL compared to peak levels of insulin in 7 week old db/db mice at around 10 ng/mL and insulin levels in non-diabetic lean controls average around 2-5 ng/mL. Results are shown in Figure 1.

Co-administration of Compound 102 with insulin enhanced the insulin blood glucose lowering response. *p < 0.05; comparing insulin to insulin/Compound 102 co-administration. Small, but statistically insignificant lowering of blood glucose in the Compound 102 treatment group is expected as a result of low level of insulin production remaining in the aged db/d/b mice.

### Example 2: Db/db Study 2

In the present study, male DbDb Lep-deficient mice (Harlan Laboratories) approximately 18 weeks of age were used. The experimental design is shown in Table 2.

**Table 2. Experimental Design Study 2**

| **Treatment** | **Insulin (dose-level; units/kg)** | **Compound 102 (dose-level; mg/kg)** | **group size** | |
|---|---|---|---|---|
| Vehicle | 0 | 0 | 6 | Blood Glucose |
| Compound 102 | 0 | 100 | 6 | Blood Glucose |
| Insulin | 1.5 | 0 | 6 | Blood Glucose |
| Compound 102/Insulin | 1.5 | 100 | 6 | Blood Glucose |

*Procedure:* Mice were divided into 4 groups of 6 mice per group. Blood glucose levels were measured using Accu-Chek Aviva Glucometers (Roche Diagnostics). The times of blood glucose levels were measured up to 72 hours after administration. Glucometers were calibrated prior to each study. Blood (5 µL) was acquired from a tail snip and directly applied to a glucose test strip. Glucose levels are reported as mg/dL.

In study 2, animals were fasted for 4 hours prior to each blood glucose measurement time point and then returned to *ad libitum* food.

Data are expressed as the average ± SEM. Data were analyzed by two-way ANOVA followed by post-hoc Bonferroni test. N=6/group

*Results:* A second study was conducted to repeat the findings of Compound 102 effects on insulin action in the 24-72 hours time window and specifically to follow the insulin / Compound 102 combination effect through until it returned to normal levels (i.e., that of vehicle-treated animals). In the second study, there were three differences from the first: 1) mice were 18 weeks of age at the time of study, 2) the insulin dose-level was reduced from 3 to 1.5 U/kg and 3) blood glucose levels were measured out until 72 hours after administration.

In this study, fasting blood glucose levels averaged 420 mg/dL. Twenty-four hours after insulin administration alone or Compound 102 administration alone, blood glucose levels were not different from vehicle controls. However, co-administration of insulin with Compound 102 elicited significant blood glucose lowering at the 24, 36 and 48 hour time points. Blood glucose levels in the insulin/Compound 102 co-administered animals returned to baseline levels by 72 hours. Results are shown in Figure 2.

Co-administration of Compound 102 with insulin enhanced the insulin blood glucose lowering response. *p < 0.05; **p < 0.01 comparing insulin to insulin/Compound 102 co-administration. The insulin treated group does not show blood glucose lowering because insulin activity normally results in its fullest extent of blood glucose lowering between 2-4 hours and is complete by 8 hours; this study did not examine that time period. The slightly elevated blood glucose in the insulin treated group at 24 and 36 hour time points is a "rebound" effect that is often observed in insulin-treated animals.

### Example 3: Streptozocoin Studies

In the present study, male CD-1: ICR mice (Charles River) approximately 8 weeks of age were used. The experimental design is shown in Table 3. Streptozocin administration to mice destroys pancreatic β-cells and is used to produce a model of Type I diabetes. In response to loss of pancreatic β-cells, mice become hyperglycemic. These mice are insulin responsive.

**Table 3. Experimental Design STZ study**

| **Treatment** | **Insulin (dose-level; units/kg)** | **Compound 102 (dose-level; mg/kg)** | **group size** | **endpoint** |
|---|---|---|---|---|
| Vehicle | 0 | 0 | 6 | Blood Glucose |
| Compound 102 | 0 | 100 | 6 | Blood Glucose |
| Insulin | 0.35 | 0 | 6 | Blood Glucose |
| Compound 102/Insulin | 0.35 | 100 | 6 | Blood Glucose |

### Procedure:

*Streptozocin administration:* Streptozocin administration to mice was conducted by a standard method. Briefly, mice were administered STZ at a dose-level of 70 mg/kg on day 1, 3 and 8. Each administration was preceded by an 18 hour fast. Mice were administered insulin (0.5 U) on days 2 and 9. On day 11, blood glucose levels were measured. Mice with blood glucose levels less than 300 mg/dL were excluded from the study. Mice were randomized to treatment groups.

Mice were tested on the insulin tolerance test on day 14 after the initial STZ administration. Mice were administered insulin (0.35U s.c.) and then 30 minutes later administered Compound 102 (100 mg/kg i.p.).

Blood glucose was measured 5, 15, 30, 60, 90, 120, 180, 240, 360, and 480 minutes after Compound 102 or vehicle administration. Blood was collected from a tail snip and drop of blood measured for glucose levels by glucometer (Accu-Chek Aviva Glucometers; Roche Diagnostics).

Data are expressed as the average ± SEM. Data were analyzed by two-way ANOVA followed by post-hoc Bonferroni test. N=6/group

*Results:* Streptozocin administration increased blood glucose levels to greater than 500 mg/dL. This level of glucose indicates that pancreatic β-cells were completely destroyed. Administration of Compound 102 did not affect blood glucose levels at any time point up to 6 hours after administration. Insulin administration reduced blood glucose levels from 546 mg/dL (baseline) to 263 mg/dL 90 minutes after administration. Blood glucose levels returned to baseline levels 4.5 hours after administration.

In combination with insulin, Compound 102 potentiated the insulin effect. Compound 102 was administered 30 minutes after insulin administration. Compound 102 increased the insulin-mediated blood glucose reduction to 157 mg/dL (compared to 263 mg/dL with insulin alone). Moreover, blood glucose levels were still reduced up to 6 hours after administration.

These data show that Compound 102 potentiates and prolongs the actions of insulin in Type I diabetic mice.

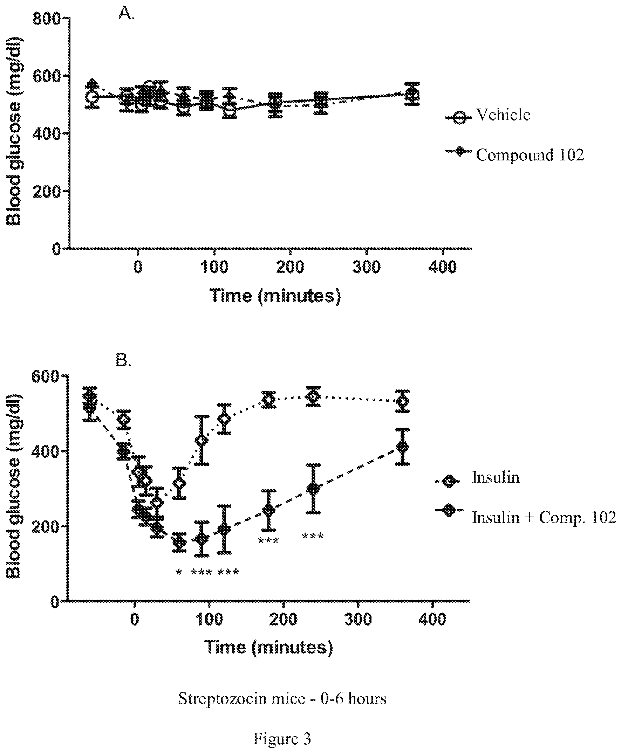

For vehicle treated and Compound 102 treatment in streptozocin Type I diabetic mice, mice were administered Compound 102 or vehicle at time 0. Blood glucose levels were measured up to 6 hours after administration. Compound 102 did not affect blood glucose levels in insulin depleted mice. For insulin in combination with Compound 102, insulin was administered 30 minutes prior to Compound 102. Compound 102 was administered at time 0. Blood glucose levels were measured up to 6 hours after administration. Administration of Compound 102 potentiated and prolonged insulin-mediated blood glucose lowering. *p<0.05 when compared to insulin alone treatment. ***p<0.001 when compared to insulin alone treatment. Data are expressed as the average ± SEM. Data were analyzed by two-way ANOVA followed by post-hoc Bonferroni test. Results are shown in Figure 3.

In these studies, co-administration of Compound 102 with insulin elicited a significantly prolonged insulin-mediated blood glucose lowering response in db/db mice in addition, administration of Compound 102 to insulin treated Type I diabetic mice potentiated and prolonged the actions of insulin.

These data suggest that Compound 102 could be used as adjunctive therapy with insulin to prolong insulin activity in late stage Type II diabetics and in Type I diabetics.

## Claims

1. A compound of Formula I: 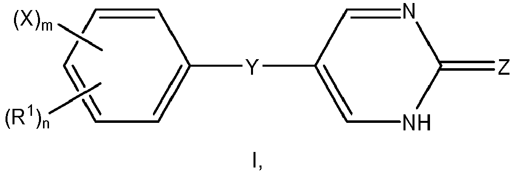 or a pharmaceutically acceptable salt thereof, wherein:
R¹ is an alkyl group;
X is a halogen;
Y is O, S, or NH;
Z is O or S; and
n is an integer from 0 to 5 and m is 0 or 1, wherein m + n is less than or equal to 5;
for use in the treatment of Type I diabetes in a mammal wherein the treatment comprises a first administration of insulin followed by administration of the compound or pharmaceutically acceptable salt thereof.

2. The compound, or pharmaceutically acceptable salt thereof, for use according to claim 1 wherein the alkyl group is methyl and n is 1.

3. The compound, or pharmaceutically acceptable salt thereof, for use according to claim 1 or claim 2, wherein the halogen is chlorine and m is 1.

4. The compound, or pharmaceutically acceptable salt thereof, for use according to any one of claims 1 to 3, wherein Y is O.

5. The compound, or pharmaceutically acceptable salt thereof, for use according to any one of claims 1 to 4, wherein Z is O.

6. The compound, or pharmaceutically acceptable salt thereof, for use according to any one of claims 1 to 5, wherein the compound is of Formula II:

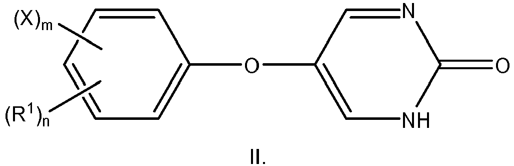

7. The compound, or pharmaceutically acceptable salt thereof, for use according to claim 1, wherein the compound is of Formula III: 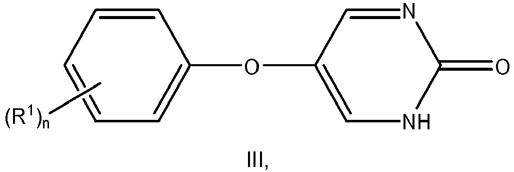 wherein R¹ is an alkyl group and n is an integer from 0 to 5.

8. The compound, or pharmaceutically acceptable salt thereof, for use according to claim 7, wherein R¹ is methyl and n is 1.

9. The compound, or pharmaceutically acceptable salt thereof, for use according to claim 8, wherein R¹ is in the meta position and the compound is of the formula:

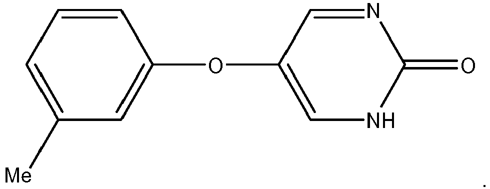

10. The compound, or pharmaceutically acceptable salt thereof, for use according to claim 1, wherein the compound is of Formula IV: 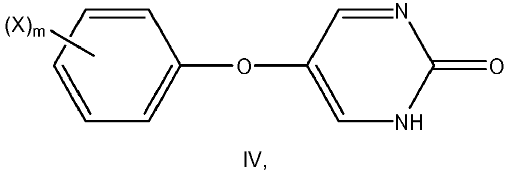 wherein X is a halogen and m is 0 or 1.

11. The compound, or pharmaceutically acceptable salt thereof, for use according to claim 10, wherein X is chlorine and m is 1.

12. The compound, or pharmaceutically acceptable salt thereof, for use according to claim 11, wherein X is in the meta position and the compound is of Formula VI:

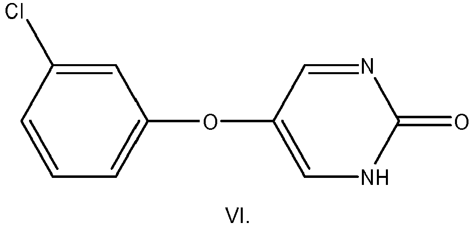

13. The compound, or pharmaceutically acceptable salt thereof, for use according to any one of claims 1 to 12, wherein the mammal is a human.

14. The compound, or pharmaceutically acceptable salt thereof, for use according to any one of claims 1 to 13, wherein the amount of the compound is from about 0.1 mg/kg to about 100 mg/kg.

15. The compound, or pharmaceutically acceptable salt thereof, for use according to any one of claims 1 to 14, wherein the insulin is selected from the group consisting of injectable insulin, transdermal insulin, and inhaled insulin.

16. The compound, or pharmaceutically acceptable salt thereof, for use according to any one of claims 1 to 14, wherein the insulin is selected from the group consisting of rapid-acting insulin, short-acting insulin, intermediate acting insulin, and long-acting insulin.

17. The compound, or pharmaceutically acceptable salt thereof, for use according to claim 16, wherein the amount of the rapid-acting insulin or short-acting insulin is from about 0.05 U/kg/day to about 5 U/kg/day, from about 0.1 U/kg/day to about 2.5 U/kg/day, or from about 0.3 U/kg/day to about 0.8 U/kg/day.

18. The compound, or pharmaceutically acceptable salt thereof, for use according to claim 16, wherein the amount of the intermediate acting insulin or long-acting insulin is from about 0.01 U/kg/day to about 3 U/kg/day, from about 0.05 U/kg/day to about 0.6 U/kg/day, or from about 0.1 U/kg/day to about 0.3 U/kg/day.

## Patentansprüche

1. Verbindung der Formel I: 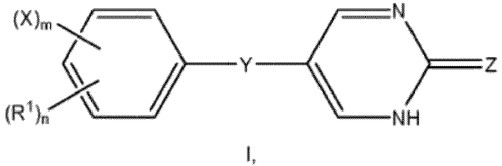 oder ein pharmazeutisch akzeptables Salz davon, wobei:
R¹ eine Alkylgruppe ist;
X ein Halogen ist;
Y O, S oder NH ist;
Z O oder S ist;
n eine ganze Zahl von 0 bis 5 ist und m 0 oder 1 ist, wobei m + n kleiner oder gleich 5 ist;
zur Verwendung bei der Behandlung von Typ-I-Diabetes bei einem Säuger, wobei die Behandlung eine erste Verabreichung von Insulin, gefolgt von der Verabreichung der Verbindung oder eines pharmazeutisch akzeptablen Salzes davon umfasst.

2. Verbindung oder pharmazeutisch akzeptables Salz davon zur Verwendung nach Anspruch 1, wobei die Alkylgruppe Methyl ist und n 1 ist.

3. Verbindung oder pharmazeutisch akzeptables Salz davon zur Verwendung nach Anspruch 1 oder Anspruch 2, wobei das Halogen Chlor ist und m 1 ist.

4. Verbindung oder pharmazeutisch akzeptables Salz davon zur Verwendung nach einem der Ansprüche 1 bis 3, wobei Y 0 ist.

5. Verbindung oder pharmazeutisch akzeptables Salz davon zur Verwendung nach einem der Ansprüche 1 bis 4, wobei Z 0 ist.

6. Verbindung oder pharmazeutisch akzeptables Salz davon zur Verwendung nach einem der Ansprüche 1 bis 5, wobei die Verbindung die Formel II hat:

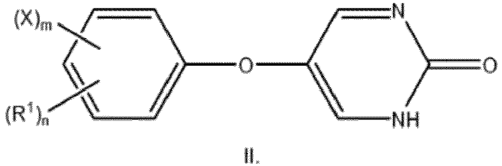

7. Verbindung oder pharmazeutisch akzeptables Salz davon zur Verwendung nach Anspruch 1, wobei die Verbindung die Formel III hat: 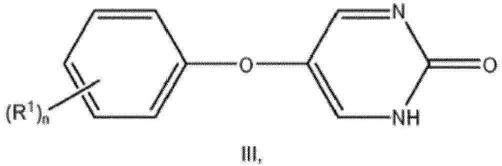 wobei R¹ eine Alkylgruppe ist und n eine ganze Zahl von 0 bis 5 ist.

8. Verbindung oder pharmazeutisch akzeptables Salz davon zur Verwendung nach Anspruch 7, wobei R¹ Methyl ist und n 1 ist.

9. Verbindung oder pharmazeutisch akzeptables Salz davon zur Verwendung nach Anspruch 8, wobei R¹ sich in der Metaposition befindet und die Verbindung die Formel: 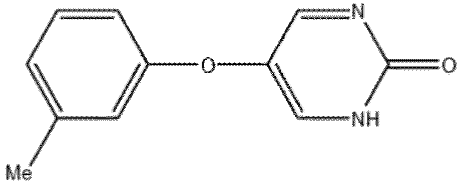 hat.

10. Verbindung oder pharmazeutisch akzeptables Salz davon zur Verwendung nach Anspruch 1, wobei die Verbindung die Formel IV hat: 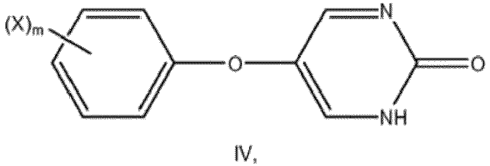 wobei X ein Halogen ist und m 0 oder 1 ist.

11. Verbindung oder pharmazeutisch akzeptables Salz davon zur Verwendung nach Anspruch 10, wobei X Chlor ist und m 1 ist.

12. Verbindung oder pharmazeutisch akzeptables Salz davon zur Verwendung nach Anspruch 11, wobei X sich in der Metaposition befindet und die Verbindung die Formel VI hat:

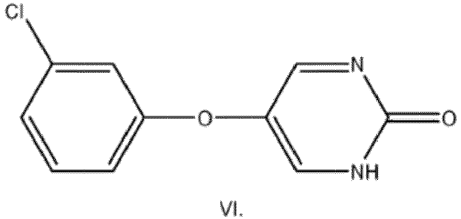

13. Verbindung oder pharmazeutisch akzeptables Salz davon zur Verwendung nach einem der Ansprüche 1 bis 12, wobei das Säugetier ein Mensch ist.

14. Verbindung oder pharmazeutisch akzeptables Salz davon zur Verwendung nach einem der Ansprüche 1 bis 13, wobei die Menge der Verbindung von etwa 0,1 mg/kg bis etwa 100 mg/kg reicht.

15. Verbindung oder pharmazeutisch akzeptables Salz davon zur Verwendung nach einem der Ansprüche 1 bis 14, wobei das Insulin ausgewählt ist aus der Gruppe bestehend aus injizierbarem Insulin, transdermalem Insulin und inhaliertem Insulin.

16. Verbindung oder pharmazeutisch akzeptables Salz davon zur Verwendung nach einem der Ansprüche 1 bis 14, wobei das Insulin ausgewählt ist aus der Gruppe bestehend aus schnell wirkendem Insulin, kurzfristig wirkendem Insulin, mittelfristig wirkendem Insulin und langfristig wirkendem Insulin.

17. Verbindung oder pharmazeutisch akzeptables Salz davon zur Verwendung nach Anspruch 16, wobei die Menge an schnell wirkendem Insulin oder kurzfristig wirkendem Insulin von etwa 0,05 U/kg/Tag bis etwa 5 U/kg/Tag, von etwa 0,1 U/kg/Tag bis etwa 2,5 U/kg/Tag oder von etwa 0,3 U/kg/Tag bis etwa 0,8/kg/Tag reicht.

18. Verbindung oder pharmazeutisch akzeptables Salz davon zur Verwendung nach Anspruch 16, wobei die Menge an mittelfristig wirkendem Insulin oder langfristig wirkendem Insulin von etwa 0,01 U/kg/Tag bis etwa 3 U/kg/Tag, von etwa 0,05 U/kg/Tag bis etwa 0,6 U/kg/Tag oder von etwa 0,1 U/kg/Tag bis etwa 0,3/kg/Tag reicht.

## Revendications

1. Composé de Formule I : 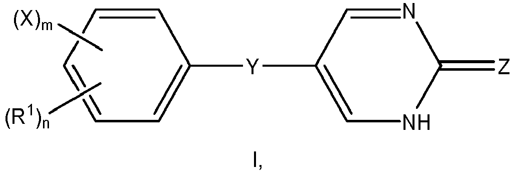 ou sel pharmaceutiquement acceptable de celui-ci, où :
R¹ représente un groupement alkyle ;
X représente un atome d'halogène ;
Y représente 0, S ou NH ;
Z représente 0 ou S ; et
n représente un entier compris entre 0 et 5 et m est égal à 0 ou à 1, où m + n est inférieur ou égal à 5 ;
pour utilisation dans le traitement du diabète de Type I chez un mammifère, où le traitement comprend une première administration d'insuline suivie par une administration du composé ou du sel pharmaceutiquement acceptable de celui-ci.

2. Composé ou sel pharmaceutiquement acceptable de celui-ci pour utilisation selon la revendication 1, où le groupement alkyle est un groupement méthyle et n est égal à 1.

3. Composé ou sel pharmaceutiquement acceptable de celui-ci pour utilisation selon la revendication 1 ou la revendication 2, où l'atome d'halogène est le chlore et m est égal à 1.

4. Composé ou sel pharmaceutiquement acceptable de celui-ci pour utilisation selon l'une quelconque des revendications 1 à 3, où Y représente 0.

5. Composé ou sel pharmaceutiquement acceptable de celui-ci pour utilisation selon l'une quelconque des revendications 1 à 4, où Z représente 0.

6. Composé ou sel pharmaceutiquement acceptable de celui-ci, pour utilisation selon l'une quelconque des revendications 1 à 5, où le composé répond à la Formule II :

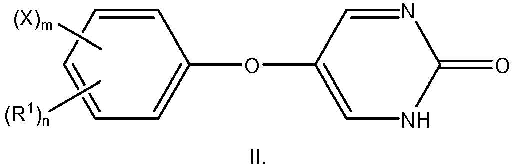

7. Composé ou sel pharmaceutiquement acceptable de celui-ci, pour utilisation selon la revendication 1, où le composé répond à la Formule III : 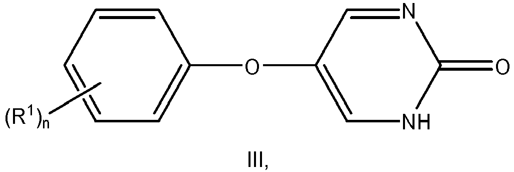 où R¹ représente un groupement alkyle et n représente un entier compris entre 0 et 5.

8. Composé ou sel pharmaceutiquement acceptable de celui-ci pour utilisation selon la revendication 7, où R¹ représente un groupement méthyle et n est égal à 1.

9. Composé ou sel pharmaceutiquement acceptable de celui-ci, pour utilisation selon la revendication 8, où R¹ est en position méta et le composé répond à la formule :

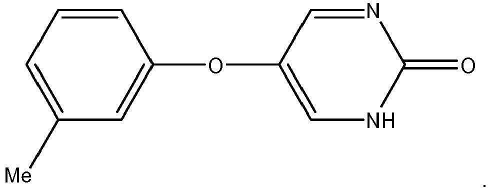

10. Composé ou sel pharmaceutiquement acceptable de celui-ci, pour utilisation selon la revendication 1, où le composé répond à la Formule IV : 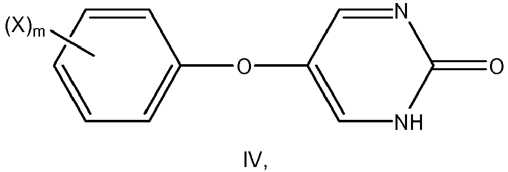 où X représente un atome d'halogène et m est égal à 0 ou à 1.

11. Composé ou sel pharmaceutiquement acceptable de celui-ci pour utilisation selon la revendication 10, où X représente un atome de chlore et m est égal à 1.

12. Composé ou sel pharmaceutiquement acceptable de celui-ci, pour utilisation selon la revendication 11, où X est en position méta et le composé répond à la Formule VI :

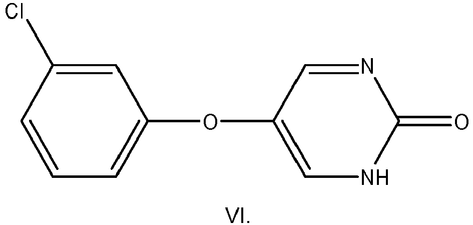

13. Composé ou sel pharmaceutiquement acceptable de celui-ci pour utilisation selon l'une quelconque des revendications 1 à 12, où le mammifère est un humain.

14. Composé ou sel pharmaceutiquement acceptable de celui-ci pour utilisation selon l'une quelconque des revendications 1 à 13, où la quantité du composé est comprise entre environ 0,1 mg/kg et environ 100 mg/kg.

15. Composé ou sel pharmaceutiquement acceptable de celui-ci pour utilisation selon l'une quelconque des revendications 1 à 14, où l'insuline est choisie dans le groupe constitué par l'insuline injectable, l'insuline transdermique et l'insuline inhalée.

16. Composé ou sel pharmaceutiquement acceptable de celui-ci pour utilisation selon l'une quelconque des revendications 1 à 14, où l'insuline est choisie dans le groupe constitué par l'insuline à action rapide, l'insuline à action courte, l'insuline à action intermédiaire et l'insuline à action longue.

17. Composé ou sel pharmaceutiquement acceptable de celui-ci pour utilisation selon la revendication 16, où la quantité de l'insuline à action rapide ou de l'insuline à action courte est comprise entre environ 0,05 U/kg/jour et environ 5 U/kg/jour, entre environ 0,1 U/kg/jour et environ 2,5 U/kg/jour, ou entre environ 0,3 U/kg/jour et environ 0,8 U/kg/jour.

18. Composé ou sel pharmaceutiquement acceptable de celui-ci pour utilisation selon la revendication 16, où la quantité de l'insuline à action intermédiaire ou de l'insuline à action longue est comprise entre environ 0,01 U/kg/jour et environ 3 U/kg/jour, entre environ 0,05 U/kg/jour et environ 0,6 U/kg/jour, ou entre environ 0,1 U/kg/jour et environ 0,3 U/kg/jour.
